# EUROPEAN PATENT SPECIFICATION

(11) **EP 3 054 909 B1**
(45) Date of publication and mention of the grant of the patent: **27.05.2026**
(21) Application number: 14799353.9
(22) Date of filing: 08.10.2014
(51) Int. Cl.: A61F 13/84, A61F 13/00

(54) **PH INDICATOR DRESSING**
WUNDVERBAND MIT PH-INDIKATOR
PANSEMENT INDICATEUR DE PH

(30) Priority: 08.10.2013 GB 201317742
(43) Date of publication of application: 17.08.2016
(73) Proprietor: Smith & Nephew plc, Watford, Hertfordshire WD18 8YE (GB)
(72) Inventor: HICKS, John, Kenneth, York YO10 5DF (GB); HAMMOND, Victoria, Jody, York YO10 5DF (GB); RICHARDSON, Mark, York YO10 5DF (GB); MCCULLOCH, Dorothy, York YO10 5DF (GB); HARTWELL, Edward, Yerbury, York YO10 5DF (GB); SAXBY, Carl, York YO10 5DF (GB)
(74) Representative: Appleyard Lees IP LLP
(86) International application number: PCT/EP2014/071510
(87) International publication number: WO 2015/052219

(56) References cited:
- WO-A1-83/00742
- WO-A2-2006/133430
- US-A- 4 813 942
- US-A- 5 181 905
- US-A1- 2004 044 299
- US-A1- 2007 161 937
- US-A1- 2007 203 442
- US-A1- 2013 066 285
- US-A1- 2013 066 289
- US-B1- 6 482 491

## Description

### Background

The field of wound care management has long understood that the pH of a wound can be an indication of wound healing status and can indicate when further action may be necessary to aid wound healing. The pH can affect many factors including oxygen release, angiogenesis, protease activity and bacterial toxicity. Acute and chronic wounds with an elevated alkaline pH have been shown to have lower rates of healing than wounds in which the pH is closer to neutral. For example, if a chronic wound has a pH of between 6 to 7.5 this indicates that wound healing is progressing well. In comparison, if the pH is between 7.5 and 8, this indicates that the wound should be monitored and a pH of above 8 indicates that clinical intervention is required. It is therefore important to be able to monitor wound pH in order to be able to assess wound healing and intervene, if necessary.

Some current wound dressings utilize a pH indicator dye provided on a colour strip integrated within the dressing. The dye changes colour (e.g., from yellow to purple) if the pH value is between 6.5 and 8.5, an indication of an infected wound. The dye is not sufficiently sensitive to provide an indication of the incremental change in pH between 6.5 and 8.5. Additionally, the dye does not provide an indication of the pH at the wound surface but rather the pH of the wound exudate at the point in the dressing where it is measured. As the pH of wound exudate can be affected by numerous external factors, including the composition of the dressing itself, the measurement of the pH of wound exudate at any significant distance away from the wound surface is often inaccurate. Whilst a pH probe allows direct measurement of the pH at the wound surface, its use can result in tissue disruption and localised cell death, such that the probe requires regular calibration. Moreover, the measurement only provides a snap-shot of the pH of a specific area of the wound at a single point in time and provides no indication of the pH changes over time. US 2004/044299 Al discloses a device for determining the pH of a wound exudate comprising a surface configured to contact the wound and a pH indicator applied to the surface.

### Summary

This application discloses devices and methods related to wound dressings having pH indicators for monitoring the pH at the wound surface. The disclosure also includes dressings in which the user is able to visually monitor incremental changes in the pH of the wound over time, using a pH scale. To monitor the pH, the pH indicator changes visually (e.g., by changing colour) as a function of the pH of the wound exudate. This provides a visual indication of the wound's pH status and enables an assessment to be made as to whether any therapeutic intervention is required in order to facilitate healing. Other advantages and improvements will be apparent to one of skill in the art upon review of the application.

A device is provided for determining pH. The device includes a surface configured to contact the wound and a pH indicator applied to the surface, wherein the pH indicator has a first colour prior to contact with the wound exudate and changes colour along a colour spectrum as a function of the pH of the wound exudate. The pH indicator changes colour in response to change in pH and this colour change is detectable at, for example, intervals of about a 0.1unit, about 0.2 unit, about 0.3 unit about 0.4 unit or about 0.5 unit interval of pH. It is envisaged that the detection level will vary based on the type of detection means utilised. For example, an electronic detector such as a colour meter, has the capability to detect a 0.1 unit change in pH. In comparison, the human eye is only capable of visually detecting a colour change which is associated with about a 0.5 unit change in pH. In a wound care setting, wound exudate pH can be detected across a broad range. In embodiments, the pH indicator utilised in the device is able to detect the pH between about pH 5 and about pH 10 and indicates changes in pH by way of a colour change along a colour spectrum, with each colour in the spectrum being associated with a particular pH. In embodiments, the pH indicator is able to detect wound pH between about pH 5.5 and about pH 9.5. More particularly the pH indicator is able to detect wound pH between about pH 6.5 and about pH 9.5. Suitable pH indicators include phenylazo compounds such as those listed in Table 1 which are available from Fraunhofer EMFT, Germany.

**Table 1: Phenylazo compounds**

| Code | Chemical name |
|---|---|
| GJM-514 | 2-[4(2-hydroxyethylsulfonyl)-phenyl]diazenyl]-4-methylphenol |
| GJM-546 | 1-hydroxy-4-[4[(hydroxyethylsulphonyl)-phenylazo]-napthalene-2-sulphonate |
| GJM-492 | 2-fluoro-4-[4[(2-hydroxyethanesulphonyl)-phenylazo]-6-methoxy phenol |
| GJM-534 | 4-[4-(2-hydroxyethylsulphonyl)-phenylazo]-2,,6-dimethoxyphenol |

The pH indicator comprises a combination of compounds which allows a broader pH range to be detected than can be detected by use of a single compound. The pH indicator comprises a combination of phenylazo compounds. In embodiments, the combination comprises at least two phenylazo compounds selected from the group listed in Table 1. In embodiments the combination comprises at least three phenylazo compounds selected from the group listed in Table 1. In embodiments, the combination comprises at least one phenylazo compound selected from the group listed in Table 1 and at least one compound that is not a phenylazo compound. In embodiments, derivatives or modifications of the phenylazo compounds listed in Table 1 are envisaged.

In embodiments, the device is a conformable, non-woven mesh or perforated film. The device can be provided in a range of sizes suitable to fit or cover a wound. Alternatively, the device can be cut to fit or cover the wound. Devices which fit or cover the wound enable the pH to be mapped across the wound rather than at selected locations. This is particularly advantageous as the pH of the wound is often not uniform across the wound. In alternative embodiments, the device can be used in isolation and placed in the wound between dressings changes in order to detect the pH of the wound. For example, the device can be incorporated into a dipstick format that can be placed into the wound between dressing changes. Alternatively, the device can be used in conjunction with a secondary wound dressing of the clinician's choice. In that scenario, the pH is assessed upon application to and removal of the device/secondary dressing from the wound. In embodiments, the device is positioned at or near a lower surface of the dressing. In certain embodiments, the device is the wound contacting layer of the secondary dressing.

In another aspect, a wound dressing is provided with the device for determining the pH of a wound exudate. The pH indicator preferably includes: (a) a wound-contacting surface, (b) an opposing non-wound contacting surface, (c) a pH indication zone comprising a pH indicator which indicates the pH of a wound exudate, wherein the colour of the pH indicator changes in response to a change in the pH of the wound exudate, and (d) at least one conduit for directing wound exudate towards the pH indication zone. The conduit helps direct wound exudate toward the pH indicator without materially altering the exudate pH en route to the indicator. In certain embodiments, the material of the conduit contains no acid or base functionality, that is to say, it is neutral and can not remove any acid or base entities from the exudate until it reaches the pH indicating system. In certain embodiments, the wound dressing has an outer surface and the pH indication zone is located at or near the outer surface. In other embodiments, the dressing has a peripheral edge extending between the wound-contacting surface and the opposing non-wound contacting surface and pH indication zone is located at or near to this peripheral edge. In certain embodiments, the conduit directs wound exudate laterally towards the pH indication zone. In embodiments, the pH indicator changes colour in response to change in pH and this colour change is detectable at, for example, intervals of about a 0.1unit, about 0.2 unit, about 0.3 unit about 0.4 unit or about 0.5 unit interval of pH. It is envisaged that the detection level will vary based on the type of detection means utilised. For example, an electronic detector such as a colour meter has the capability to detect a 0.1 unit change in pH. In comparison, the human eye is only capable of visually detecting a colour change which is associated with about a 0.5 unit change in pH. In a wound care setting, wound exudate pH can be detected across a broad range. In embodiments, the pH indicator utilised in the device is able to detect the pH between pH 5 and 10 and indicates changes in pH by way of a colour change along a colour spectrum, with each colour in the spectrum being associated with a particular pH. In embodiments, the pH indicator is able to detect wound pH between about pH 5 and about pH10. Particularly, the pH indicator is able to detect wound pH between about pH 5.5 and about pH 9.5. More particularly, the pH indicator is able to detect wound pH between about pH 6.5 and about pH 9.5. Suitable pH indicators include phenylazo compounds such as those selected from the group listed in Table 1. The pH indicator comprises a combination of compounds which allows a broader pH range to be detected than can be detected by use of a single compound. The pH indicator comprises a combination of phenylazo compounds. In embodiments, the combination comprises at least two phenylazo compounds selected from the group listed in Table 1. In embodiments, the combination comprises at least three phenylazo compounds selected from the group listed in Table 1. In embodiments, the combination comprises at least one phenylazo compound selected from the group listed in Table 1 and at least one compound that is not a phenylazo compound. In embodiments, derivatives or modifications of the phenylazo compounds listed in Table 1 are envisaged.

Further areas of applicability of the disclosed devices and methods will become apparent from the detailed description provided hereinafter. It should be understood that the detailed description and specific examples, while indicating particular embodiments, are intended for purposes of illustration only and are not intended to limit the scope of the disclosure or any of the claims that may be pursued.

### Description of the drawings

The foregoing and other objects and advantages will be appreciated more fully upon consideration of the following detailed description, taken in conjunction with the accompanying drawings, in which like reference numbers refer to like parts throughout. The invention is defined in the appended claims. Any disclosure lying outside the scope of said claims is only intended for illustrative as well as comparative purposes. These depicted embodiments are to be understood as illustrative and not limiting in any way:
Figure 1 illustrates an embodiment of a negative pressure system.
Figure 2 is a schematic illustration of a system for the treatment of abdominal wounds.
Figure 3 illustrates an embodiment of a wound treatment system.
Figures 4A-D illustrate the use and application of an embodiment of a wound treatment system onto a patient which may be used with any dressing embodiment disclosed herein.
Figure 5 illustrates an embodiment of a negative pressure wound treatment system employing a wound dressing capable of absorbing and storing wound exudate and a flexible suction adapter.
Figure 6 illustrates another embodiment of a wound dressing in cross-section.
Figure 7 illustrates an exploded view of another embodiment of a wound dressing.
Figures 8A and 8B are side cross-sectional views of an illustrative device having a pH indicator, the colour of which changes as a result of alterations in the pH of the wound exudate.
Figures 9A-D illustrate side cross-sectional views of an illustrative wound dressing having a device shown in Figures 8A and 8B applied to is wound-facing surface.
Figures 10A and 10B are side cross-sectional views of an illustrative wound dressing in which wound exudate is guided via a conduit to a pH indication zone which includes a pH indicator, the colour of the indicator changes as a result of alterations in the pH of the wound exudate.
Figure 11 is a photograph of a Post-Op sample dyed with GJM-514, illustrating changes in colour of the dye in response to solution changing pH along a pH unit interval scale.
Figures 12A-F are graphic representations of colour pen measurements for the Post-Op sample illustrated in Figure 11.
Figure 13 is a photograph of a Post-Op sample dyed with a first combination of dyes, illustrating changes in colour of the dye combination in response to a solution changing pH along a pH unit interval scale.
Figures 14A-D are graphic representations of colour pen measurements for the Post-Op sample illustrated in Figure 13.
Figure 15 is a photograph of a Post-Op sample dyed with a second combination of dyes, illustrating changes in colour of the dye combination in response to a buffered solution changing pH along a pH unit interval scale.
Figures 16A-E are graphic representations of the colour pen measurements for the Post-Op sample illustrated in Figure 15.
Figure 17 is a photograph of a Post-Op sample dyed with a third combination of dyes, illustrating changes in colour of the dye combination in response to a buffered solution changing pH along a pH unit interval scale.
Figures 18A-F are graphic representations of the colour pen measurements for the Post-Op sample illustrated in Figure 17.
Figure 19 is a photograph of a Post-Op sample dyed with a fourth combination of dyes, illustrating changes in colour of the dye combination in response to a buffered solution changing pH along a pH unit interval scale.
Figures 20A-E are graphic representations of the colour pen measurements for the Post-Op sample illustrated in Figure 19.
Figures 21 A-F are photographs of pH sensitive gauze in ex-vivo wound model with alternating pH 5 and pH 8 horse serum being pumped in.

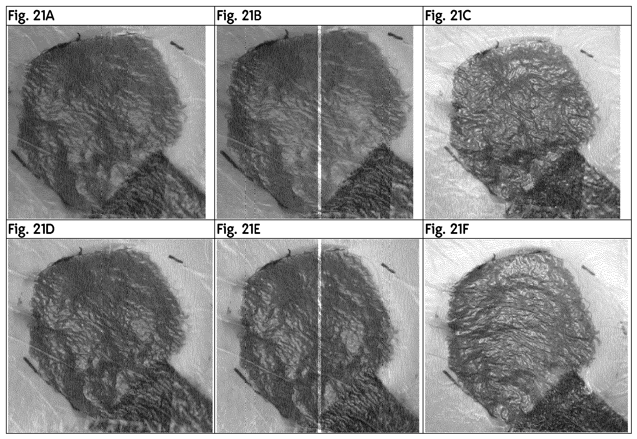

Figure 21A is a photograph of pH5 after 2.5 hours approx.
Figure 21B is a photograph of pH5 after 5.5 hours approx.
Figure 21C is a photograph of pH 8 after 15 hours approx.
Figure 21D is a photograph of pH 5 after 3.5 hours approx.
Figure 21E is a photograph of pH 5 after 5.5 hours approx. with the flow rate of horse serum increased at 3.5 hours.
Figure 21F is a photograph of pH 5 after 7.5 hours with the flow rate of horse serum increased at 3.5 hours and at 5.5 hours.

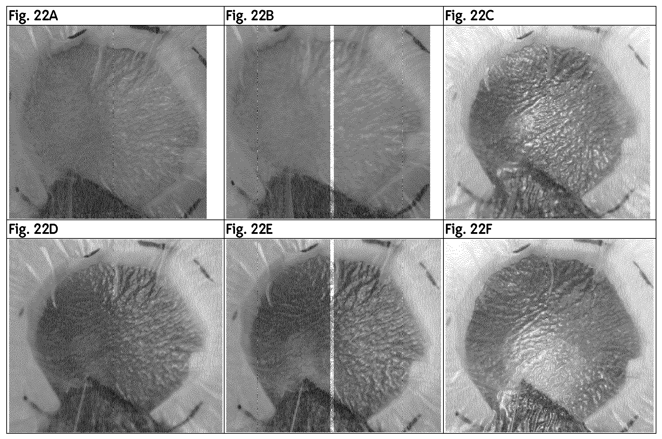

Figures 22A to F are photographs of pH sensitive foam (V.A.C. WhiteFoam trade mark of KCI) in an ex-vivo wound model with alternating pH5 and pH 8 horse serum being pumped in.
Figure 22A is a photograph at pH 5 after 2.5 hours approx..
Figure 22B is a photograph at pH 5 after 5.5 hours approx..
Figure 22C is a photograph at pH 8 after 15 hours approx..
Figure 22D is a photograph at pH 5 after 3.5 hours approx..
Figure 22E is a photograph at pH 5 after 5.5 hours approx.., with the flow rate of horse serum increased at 3.5 hours.
Figure 22F is a photograph at pH 5 after 7.5 hours approx.., with the flow rate of horse serum increased at 3.5 hours and at 5.5 hours.

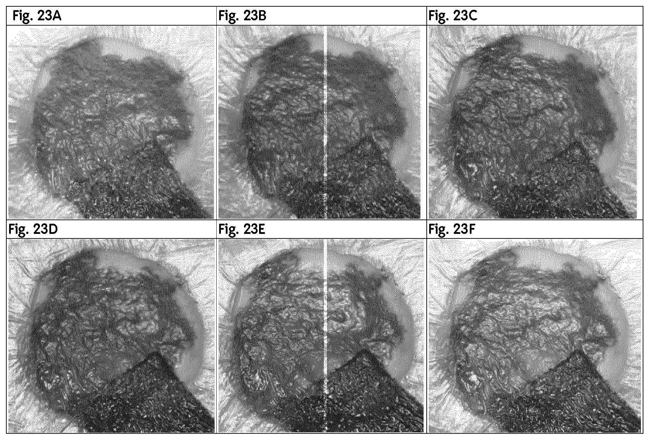

Figures 23 A to E are photographs of pH sensitive gauze in an ex-vivo wound model with alternating basic and acidic water being pumped in.
Figure 23A is a photograph at 8am Day 1 showing basic pH.
Figure 23B is a photograph at 12:57 pm Day 1 (5 hours) showing basic pH.
Figure 23C is a photograph at 08:03am Day 2 (24 hours) showing basic pH.
Figure 23D is a photograph at 12:41pm Day 2 (5 hours) showing acidic pH.
Figure 23E is a photograph at 15:06 Day 2 (7 hours) showing acidic pH.
Figure 23F is a photograph at 16:47 Day 2 (9 hours) showing acidic pH.

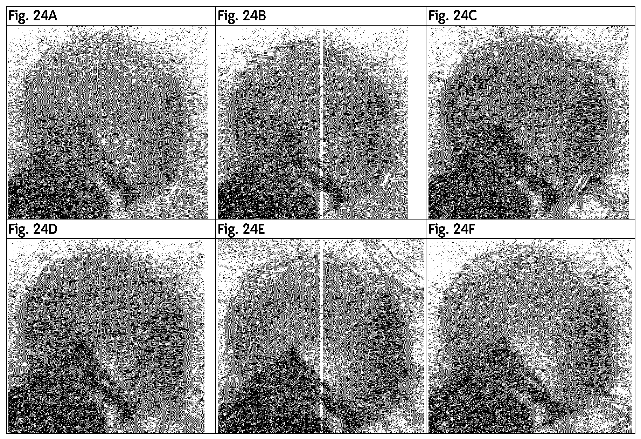

Figures 24A to F are photographs of pH sensitive foam in an ex-vivo wound model with alternating basic and acidic water being pumped in.
Figure 24A is a photograph at 8am Day 1 showing basic pH.
Figure 24B is a photograph at 12:57 pm Day 1 (5 hours) showing basic pH.
Figure 24C is a photograph at 08:03 am Day 2 (24 hours) showing basic pH.
Figure 24D is a photograph at 09:06 Day 2 (1 hour) showing acidic pH.
Figure 24E is a photograph at 15:06 Day 2 (7 hours) showing acidic pH.
Figure 24F is a photograph at 16:47 Day 2 (9 hours) showing acidic pH.

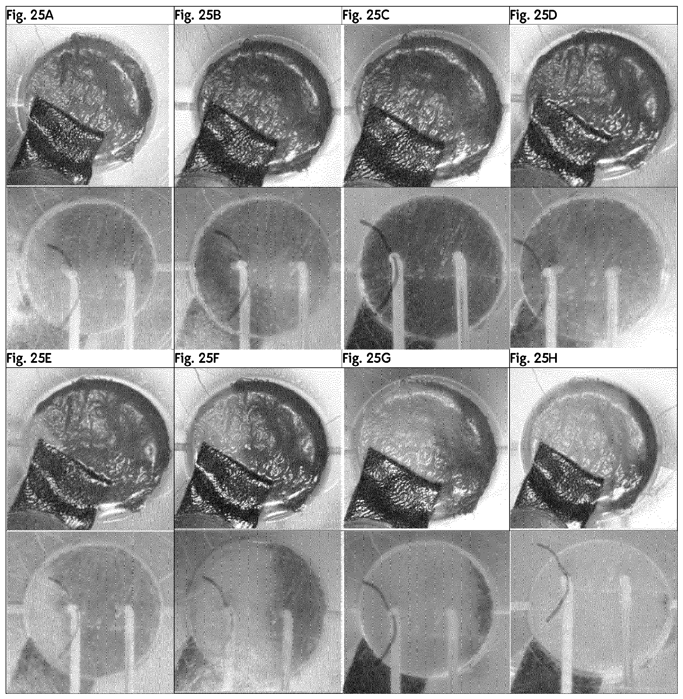

Figures 25A to H are photographs of pH sensitive foam in a clear Perspex wound model with alternating basic and acidic water.
Figure 25A is a photograph at 8am Day 1 showing basic pH.
Figure 25B is a photograph at 12:56 Day 1 (5 hours) showing basic pH.
Figure 25C is a photograph at 16:20 Day 1 (8.5 hours) showing basic pH.
Figure 25D is a photograph at 8:02 am Day 2 (24 hours) showing basic pH.
Figure 25E is a photograph at 09:05 am Day 2 (1 hour) showing acidic pH.
Figure 25F is a photograph at 10:50am Day 2 (3 hours) showing acidic pH.
Figure 25G is a photograph at 13:26 Day 2 (5.5 hours) showing acidic pH.
Figure 25H is a photograph at 15:05 Day 2 (7 hours)showing acidic pH.

### Detailed Description

To provide an understanding of the devices and methods describe herein, certain illustrative embodiments and examples will now be described.

Embodiments disclosed herein relate to apparatuses and methods to treat and/or evaluate a wound, including pump components, wound dressing components, and apparatuses that incorporate one or more pH indicators and may be used to apply negative pressure wound therapy. The apparatuses and components comprising the wound overlay and packing materials, if any, are sometimes collectively referred to herein as dressings.

It will be appreciated that throughout this specification reference is made to a wound. It is to be understood that the term wound is to be broadly construed and encompasses open and closed wounds in which skin is torn, cut or punctured or where trauma causes a contusion, or any other superficial or other conditions or imperfections on the skin of a patient or otherwise that benefit from reduced pressure treatment. A wound is thus broadly defined as any damaged region of tissue where fluid may or may not be produced. Examples of such wounds include, but are not limited to, abdominal wounds or other large or incisional wounds, either as a result of surgery, trauma, sterniotomies, fasciotomies, or other conditions, dehisced wounds, acute wounds, chronic wounds, subacute and dehisced wounds, traumatic wounds, flaps and skin grafts, lacerations, abrasions, contusions, burns, diabetic ulcers, pressure ulcers, stoma, surgical wounds, trauma and venous ulcers or the like.

Some of the wound dressings described herein may be used as part of a negative pressure or reduced pressure system. As is used herein, reduced or negative pressure levels, such as -X mmHg, represent pressure levels that are below standard atmospheric pressure, which corresponds to 760 mmHg (or 1 atm, 29.93 inHg, 101.325 kPa, 14.696 psi, etc.). Accordingly, a negative pressure value of -X mmHg reflects absolute pressure that is X mmHg below 760 mmHg or, in other words, an absolute pressure of (760-X) mmHg. In addition, negative pressure that is "less" or "smaller" than X mmHg corresponds to pressure that is closer to atmospheric pressure (e.g., -40 mmHg is less than -60 mmHg). Negative pressure that is "more" or "greater" than -X mmHg corresponds to pressure that is further from atmospheric pressure (e.g., -80 mmHg is more than -60 mmHg).

The negative pressure range for some embodiments of the present disclosure can be approximately -80 mmHg, or between about -20 mmHg and -200 mmHg. Note that these pressures are relative to normal ambient atmospheric pressure. Thus, -200 mmHg would be about 560 mmHg in practical terms. In some embodiments, the pressure range can be between about -40 mmHg and -150 mmHg. Alternatively a pressure range of up to -75 mmHg, up to -80 mmHg or over -80 mmHg can be used. Also in other embodiments a pressure range of below - 75 mmHg can be used. Alternatively, a pressure range of over approximately -100 mmHg, or even -150 mmHg, can be supplied by the negative pressure apparatus. In some embodiments, negative pressure may be varied over time for example using a sinusoidal wave, square wave, and/or in synchronization with one or more patient physiological indices (e.g., heartbeat). Examples of such applications where additional disclosure relating to the preceding may be found include Application Serial No. 11/919,355, titled "Wound treatment apparatus and method," filed October 26, 2007, published as US 2009/0306609; and U.S. Patent No. 7,753,894, titled "Wound cleansing apparatus with stress," issued July 13, 2010. Other applications that may contain teachings relevant for use with the embodiments described herein may include Application Serial No. 12/886,088, titled "Systems And Methods For Using Negative Pressure Wound Therapy To Manage Open Abdominal Wounds," filed September 20, 2010, published as US 2011/0213287; Application Serial No. 13/092,042, titled "Wound Dressing And Method Of Use," filed April 21, 2011, published as US 2011/0282309.

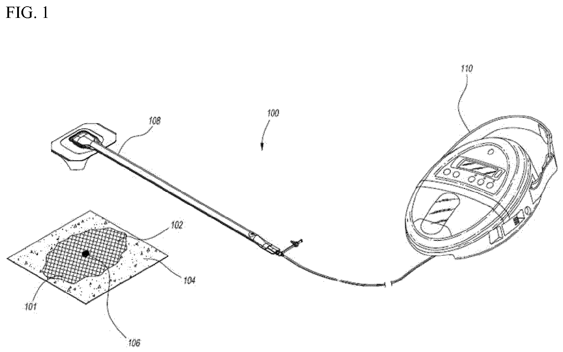

Figure 1 illustrates an embodiment of a negative pressure treatment system 100 that comprises a wound packer 102 inserted into a wound 101. The wound packer 102 may comprise porous materials such as foam, and in some embodiments may comprise one or more embodiments of wound closure devices described in further detail herein. In some embodiments, the perimeter or top of any wound closure device inserted into the wound 101 may also be covered with foam or other porous materials. A drape 104 may be placed over the wound 101, and is preferably adhered or sealed to the skin on the periphery of the wound 101 so as to create a fluid-tight seal. An aperture 106 may be made through the drape 104-which can be manually made or preformed into the drape 104-so as to provide a fluidic connection from the wound 101 to a source of negative pressure such as a pump 110. Preferably, the fluidic connection between the aperture 106 and the pump 110 is made via a conduit 108. In some embodiments, the conduit 108 may comprise a RENASYS^{®} Soft Port^{™}, manufactured by Smith & Nephew. Of course, in some embodiments, the drape 104 may not necessarily comprise an aperture 106, and the fluidic connection to the pump 110 may be made by placing the conduit 108 below the drape. In some wounds, particularly larger wounds, multiple conduits 108 may be used, fluidically connected via one or more apertures 106.

In some embodiments, the drape 104 may be provided with one or more corrugations or folds. Preferably, the corrugations are aligned along the longitudinal axis of the wound, and as such may support closure of the wound by preferentially collapsing in a direction perpendicular to the longitudinal axis of the wound. Such corrugations may aid in the application of contractile forces parallel to the wound surface and in the direction of wound closure. Examples of such drapes may be found in Application Serial No. 12/922,118, titled "Vacuum Closure Device," filed November 17, 2010 (published as US 2011/00543 65).

In use, the wound 101 is prepared and cleaned. In some cases, such as abdominal wounds, a non- or minimally-adherent organ protection layer (not illustrated) may be applied over any exposed viscera. The wound packer 102 is then inserted into the wound, and is covered with the drape 104 so as to form a fluid-tight seal. A first end of the conduit 108 is then placed in fluidic communication with the wound, for example via the aperture 106. The second end of the conduit 108 is connected to the pump 110. The pump 110 may then be activated so as to supply negative pressure to the wound 101 and evacuate wound exudate from the wound 101. As will be described in additional detail below and in relation to the embodiments of the foregoing wound closure devices, negative pressure may also aid in promoting closure of the wound 101, for example by approximating opposing wound margins. In certain embodiments, a pH indicator dye, as will be described in more detail later in the specification, may be incorporated into the wound filler to visually indicate the pH of the wound.

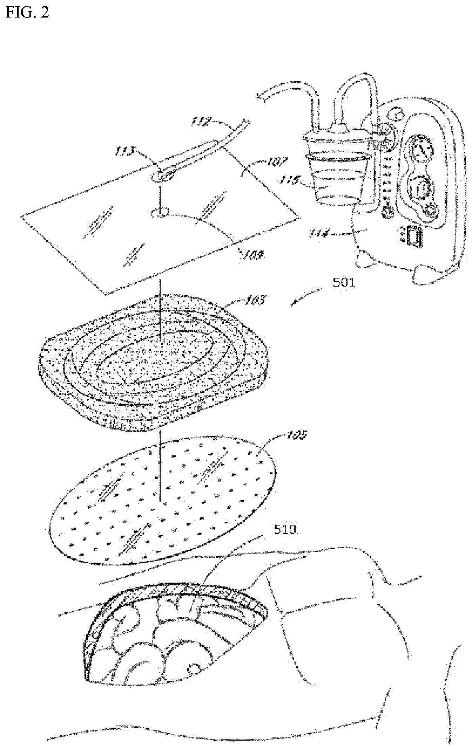

Turning to Figure 2, treatment of a wound with negative pressure in certain embodiments uses a negative pressure treatment system 501 as illustrated schematically here. In this embodiment, a wound site 510, illustrated here as an abdominal wound site, may benefit from treatment with negative pressure. Such abdominal wound sites may be a result of, for example, an accident or due to surgical intervention. In some cases, medical conditions such as abdominal compartment syndrome, abdominal hypertension, sepsis, or fluid edema may require decompression of the abdomen with a surgical incision through the abdominal wall to expose the peritoneal space, after which the opening may need to be maintained in an open, accessible state until the condition resolves. Other conditions may also necessitate that an opening-particularly in the abdominal cavity-remain open, for example if multiple surgical procedures are required (possibly incidental to trauma), or there is evidence of clinical conditions such as peritonitis or necrotizing fasciitis.

In cases where there is a wound, particularly in the abdomen, management of possible complications relating to the exposure of organs and the peritoneal space is desired, whether or not the wound is to remain open or if it will be closed. Therapy, preferably using the application of negative pressure, can be targeted to minimize the risk of infection, while promoting tissue viability and the removal of deleterious substances from the wound site. The application of reduced or negative pressure to a wound site has been found to generally promote faster healing, increased blood flow, decreased bacterial burden, increased rate of granulation tissue formation, to stimulate the proliferation of fibroblasts, stimulate the proliferation of endothelial cells, close chronic open wounds, inhibit burn penetration, and/or enhance flap and graft attachment, among other things. It has also been reported that wounds that have exhibited positive response to treatment by the application of negative pressure include infected open wounds, decubitus ulcers, dehisced incisions, partial thickness burns, and various lesions to which flaps or grafts have been attached. Consequently, the application of negative pressure to a wound site 510 can be beneficial to a patient.

Accordingly, certain embodiments provide for a wound contact layer 105 to be placed over the wound site 510. Preferably, the wound contact layer 105 can be a thin, flexible material which will not adhere to the wound site or the exposed viscera in close proximity. For example, polymers such as polyurethane, polyethylene, polytetrafluoroethylene, or blends thereof may be used. In one embodiment, the wound contact layer is permeable. For example, the wound contact layer 105 can be provided with openings, such as holes, slits, or channels, to allow the removal of fluids from the wound site 510 or the transmittal of negative pressure to the wound site 510. Additional embodiments of the wound contact layer 105 are described in further detail below. In certain embodiments, a pH indicator dye, as will be described in more detail later in the specification, may be incorporated into the wound contact layer to visually indicate the pH of the wound.

Certain embodiments of the negative pressure treatment system 101 may also use a porous pad 103, which can be disposed over the wound contact layer 105. This pad 103 can be constructed from a porous material, for example foam, that is soft, resiliently flexible, and generally conformable to the wound site 510. Such a foam can include an open-celled and reticulated foam made, for example, of a polymer. Suitable foams include foams composed of, for example, polyurethane, silicone, and polyvinyl alcohol. Preferably, this pad 103 can channel wound exudate and other fluids through itself when negative pressure is applied to the wound. Some pads 103 may include preformed channels or openings for such purposes. In certain embodiments, the pad 103 may have a thickness between about one inch and about two inches. The pad may also have a length of between about 16 and 17 inches, and a width of between about 11 and 12 inches. In other embodiments, the thickness, width, and/or length can have other suitable values. Other aspects of the pad 103 are discussed in further detail below. In some embodiments, a pH indicator dye, as will be described in more detail later in the specification, may be incorporated into the porous pad to visually indicate the pH of the wound.

Preferably, a drape 107 is used to seal the wound site 510. The drape 107 can be at least partially liquid impermeable, such that at least a partial negative pressure may be maintained at the wound site. Suitable materials for the drape 107 include, without limitation, synthetic polymeric materials that do not significantly absorb aqueous fluids, including polyolefins such as polyethylene and polypropylene, polyurethanes, polysiloxanes, polyamides, polyesters, and other copolymers and mixtures thereof. The materials used in the drape may be hydrophobic or hydrophilic. Examples of suitable materials include Transeal^{®} available from DeRoyal and OpSite^{®} available from Smith & Nephew. In order to aid patient comfort and avoid skin maceration, the drapes in certain embodiments are at least partly breathable, such that water vapor is able to pass through without remaining trapped under the dressing. An adhesive layer may be provided on at least a portion the underside of the drape 107 to secure the drape to the skin of the patient, although certain embodiments may instead use a separate adhesive or adhesive strip. Optionally, a release layer may be disposed over the adhesive layer to protect it prior to use and to facilitate handling the drape 107; in some embodiments, the release layer may be composed of multiple sections. In certain embodiments, a pH indicator dye, as will be described in more detail later in the specification, may be incorporated into the drape to visually indicate the pH of the wound.

The negative pressure system 501 can be connected to a source of negative pressure, for example a pump 114. One example of a suitable pump is the Renasys EZ pump available from Smith & Nephew. The drape 107 may be connected to the source of negative pressure 114 via a conduit 112. The conduit 112 may be connected to a port 113 situated over an aperture 109 in the drape 107, or else the conduit 112 may be connected directly through the aperture 109 without the use of a port. In a further alternative, the conduit may pass underneath the drape and extend from a side of the drape. U.S. Patent No. 7,524,315 discloses other similar aspects of negative pressure systems.

In many applications, a container or other storage unit 115 may be interposed between the source of negative pressure 114 and the conduit 112 so as to permit wound exudate and other fluids removed from the wound site to be stored without entering the source of negative pressure. Certain types of negative pressure sources-for example, peristaltic pumpsmay also permit a container 115 to be placed after the pump 114. Some embodiments may also use a filter to prevent fluids, aerosols, and other microbial contaminants from leaving the container 115 and/or entering the source of negative pressure 114. Further embodiments may also include a shut-off valve or occluding hydrophobic and/or oleophobic filter in the container to prevent overflow; other embodiments may include sensing means, such as capacitative sensors or other fluid level detectors that act to stop or shut off the source of negative pressure should the level of fluid in the container be nearing capacity. At the pump exhaust, it may also be preferable to provide an odor filter, such as an activated charcoal canister.

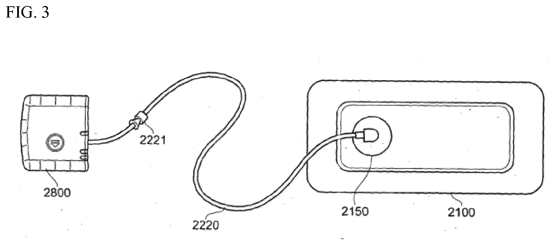

Figure 3 illustrates an embodiment of a negative pressure wound treatment comprising a wound dressing 2100 in combination with a pump 2800. As stated above, the wound dressing 2100 can be any wound dressing embodiment disclosed herein this section or elsewhere in the specification or have any combination of features of any number of wound dressing embodiments disclosed herein. Here, the dressing 2100 may be placed over a wound as described previously, and a conduit 2220 may then be connected to the port 2150, although in some embodiments the dressing 2100 may be provided with at least a portion of the conduit 2220 pre-attached to the port 2150. Preferably, the dressing 2100 is provided as a single article with all wound dressing elements (including the port 2150) pre-attached and integrated into a single unit. The wound dressing 2100 may then be connected, via the conduit 2220, to a source of negative pressure such as the pump 2800. The pump 2800 can be miniaturized and portable, although larger conventional pumps may also be used with the dressing 2100. In some embodiments, the pump 2800 may be attached or mounted onto or adjacent the dressing 2100. A connector 2221 may also be provided so as to permit the conduit 2220 leading to the wound dressing 2100 to be disconnected from the pump, which may be useful for example during dressing changes. In certain embodiments, a pH indicator dye, as will be described in more detail later in the specification, may be incorporated into the wound dressing 2100 to visually indicate the pH of the wound.

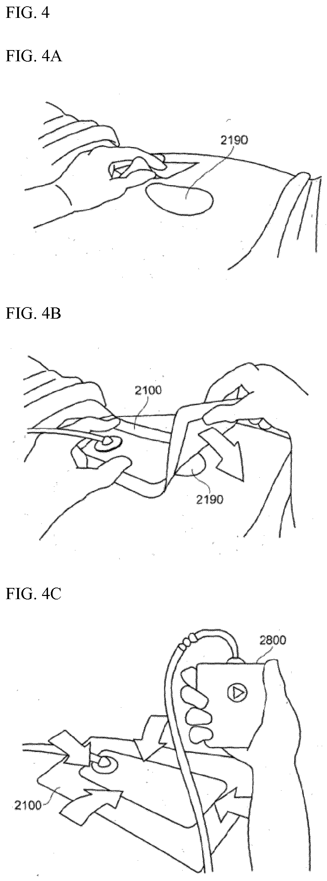

Figures 4A-D illustrate the use of an embodiment of a negative pressure wound treatment system being used to treat a wound site on a patient. Figure 4A shows a wound site 2190 being cleaned and prepared for treatment. Here, the healthy skin surrounding the wound site 2190 is preferably cleaned and excess hair removed or shaved. The wound site 2190 may also be irrigated with sterile saline solution if necessary. Optionally, a skin protectant may be applied to the skin surrounding the wound site 2190. If necessary, a wound packing material, such as foam or gauze, may be placed in the wound site 2190. This may be preferable if the wound site 2190 is a deeper wound.

After the skin surrounding the wound site 2190 is dry, and with reference now to Figure 4B, the wound dressing 2100 may be positioned and placed over the wound site 2190. Preferably, the wound dressing 2100 is placed with the wound contact layer 2102 over and/or in contact with the wound site 2190. In some embodiments, an adhesive layer is provided on the lower surface 2101 of the wound contact layer 2102, which may in some cases be protected by an optional release layer to be removed prior to placement of the wound dressing 2100 over the wound site 2190. Preferably, the dressing 2100 is positioned such that the port 2150 is in a raised position with respect to the remainder of the dressing 2100 so as to avoid fluid pooling around the port. In some embodiments, the dressing 2100 is positioned so that the port 2150 is not directly overlying the wound, and is level with or at a higher point than the wound. To help ensure adequate sealing for negative pressure wound therapy, the edges of the dressing 2100 are preferably smoothed over to avoid creases or folds. A pH indicator dye, as will be described in more detail later in the specification, is incorporated into the adhesive layer and/or the wound contact layer to visually indicate the pH of the wound.

With reference now to Figure 4C, the dressing 2100 is connected to the pump 2800. The pump 2800 is configured to apply negative pressure to the wound site via the dressing 2100, and typically through a conduit. In some embodiments, and as described above in Figure 3, a connector may be used to join the conduit from the dressing 2100 to the pump 2800. Upon the application of negative pressure with the pump 2800, the dressing 2100 may in some embodiments partially collapse and present a wrinkled appearance as a result of the evacuation of some or all of the air underneath the dressing 2100. In some embodiments, the pump 2800 may be configured to detect if any leaks are present in the dressing 2100, such as at the interface between the dressing 2100 and the skin surrounding the wound site 2190. Should a leak be found, such leak is preferably remedied prior to continuing treatment.

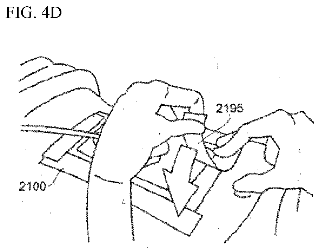

Turning to Figure 4D, additional fixation strips 2195 may also be attached around the edges of the dressing 2100. Such fixation strips 2195 may be advantageous in some situations so as to provide additional sealing against the skin of the patient surrounding the wound site 2190. For example, the fixation strips 2195 may provide additional sealing for when a patient is more mobile. In some cases, the fixation strips 2195 may be used prior to activation of the pump 2800, particularly if the dressing 2100 is placed over a difficult to reach or contoured area. Treatment of the wound site 2190 preferably continues until the wound has reached a desired level of healing. In some embodiments, it may be desirable to replace the dressing 2100 after a certain time period has elapsed, or if the dressing is full of wound fluids. During such changes, the pump 2800 may be kept, with just the dressing 2100 being changed. In certain embodiments, a pH indicator dye, as will be described in more detail later in the specification, may be incorporated into the fixation strips to visually indicate the pH of the wound.

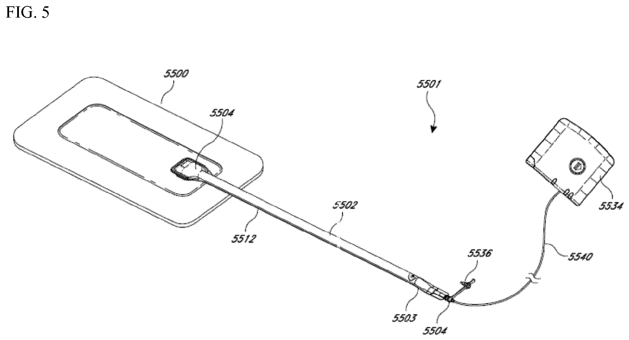

Figure 5 illustrates an embodiment of a negative pressure wound treatment system 5501 employing a wound dressing 5500 in conjunction with a flexible suction adapter 5512. The wound dressing 5500 may be similar to the dressings illustrated in Figures 3-4D. Here, the flexible suction adapter 5512 may comprise a bridge 5502 having a proximal end 5503 and a distal end 5505 and an applicator 5504 at the distal end 5505 of the bridge 5502. A connector 5504 is preferably disposed at the proximal end 5503 of the bridge 5502. A cap 5536 may be provided with the system 5501 (and can in some cases, as illustrated, be attached to the connector 5504). The cap 5536 can be useful in preventing fluids from leaking out of the proximal end 5503. The system 5501 may include a source of negative pressure such as a pump or negative pressure unit 5534 capable of supplying negative pressure. The pump optionally comprises a canister or other container for the storage of wound exudates and other fluids that may be removed from the wound. In some embodiments, the pump 5534 can be a PICO^{™} pump, as sold by Smith & Nephew, which does not include a canister, and wound exudate removed from the wound by negative pressure is retained within the wound dressing. The pump 5534 may be connected to the connector 5504 via a tube 5540. In use, the dressing 5500 is placed over a suitably-prepared wound, which may in some cases be filled with a wound packing material such as foam or gauze. Subsequently, with the pump 5534 connected via the tube 5540 to the connector 5504, the pump is activated, thereby supplying negative pressure to the wound. Application of negative pressure may be applied until a desired level of healing of the wound 5530 is achieved.

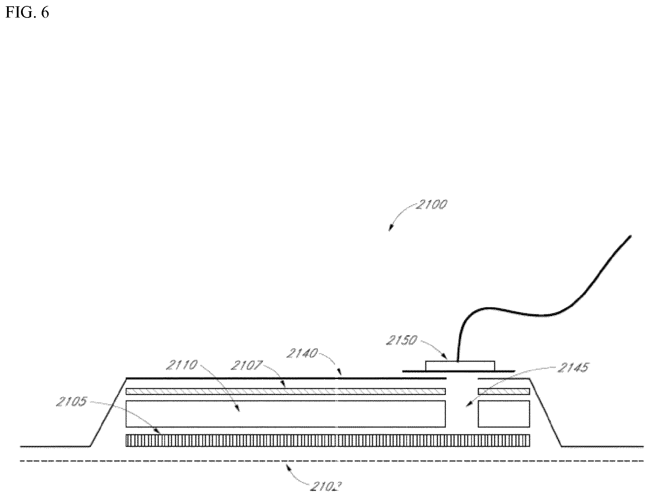

With reference now to Figure 6, which shares many of the elements illustrated in the previous figures, the embodiment of the wound dressing illustrated here comprises the backing layer 2140, an optional masking layer 2107, and absorbent layer 2110. All of these layers may optionally have a cut or opening made therethrough which communicate directly to an optional transmission layer 2105 so as to form an orifice 2145. Wound contact layer 2102 may be adhered to a lower surface of the backing layer 2140 to enclose the absorbent layer 2110. The suction port 2150 is preferably situated above an opening in the backing layer 2140 and communicates with the optional orifice 2145. A filter (not shown) may be provided on or below the suction port 2150 to prevent liquid from passing through the opening in the backing layer 2140. In certain embodiments, a pH indicator dye, as will be described in more detail later in the specification, may be incorporated into the filter to visually indicate the pH of the wound.

In particular for embodiments with a single port 2150, it may be preferable for the port 2150 to be located in an off-center position. Such a location may permit the dressing 2100 to be positioned onto a patient such that the port 2150 is raised in relation to the remainder of the dressing 2100. So positioned, the port 2150 and the filter may be less likely to come into contact with wound fluids that could prematurely occlude the filter so as to impair the transmission of negative pressure to the wound site.

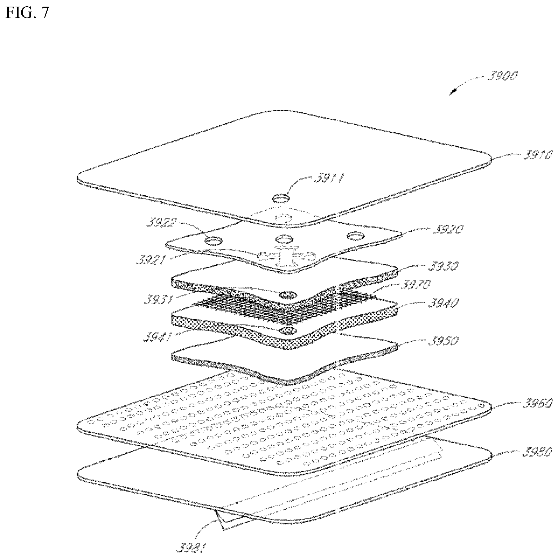

Figure 7 illustrates another embodiment of a wound dressing 3900. The wound dressing may comprise a release layer 3980, wound contact layer 3960, a transmission layer 3950, an acquisition distribution layer 3940, an adhesive layer 3970, an absorbent layer 3930, an obscuring layer 3920, and a backing layer 3910. One or more of the aforementioned layers may be optional. Although this figure illustrates a dressing having one particular shape, the construction of the layers can be applied to any of the embodiments identified herein this section or elsewhere in the specification. In certain embodiments, a pH indicator dye, as will be described in more detail later in the specification, may be incorporated into the release layer 3980, wound contact layer 3960, transmission layer 3950, acquisition distribution layer 3940, adhesive layer 3970, absorbent layer 3930, obscuring layer 3920, and/or the backing layer 3910 to visually indicate the pH of the wound. The dressing may incorporate one or more viewing windows (not shown) to allow a clinician and/or user to easily view any component within the dressing that may be impregnated with a pH indicator dye. Such viewing windows may be incorporated into any of the layers overlying a layer incorporating a pH indicator dye. By utilizing the one or more viewing windows, a clinician is then able to monitor the pH of the wound through visually observing changes in the color of the dressing component impregnated with a pH indicator dye.

The dressing 3900 may be connected to a port (not shown) provided over the opening 3911 in the backing layer 3910, such as described with respect to the above embodiments. At least the backing layer 3910, obscuring layer 3920, absorbent layer 3930, and acquisition distribution layer 3940 may optionally have openings underlying the port. In some embodiments, the opening 3921 in the obscuring layer may be cross-shaped. As illustrated, the cross-shaped opening 3921 may comprise four arms of roughly equal length extending outward from a central point of intersection of the arms, wherein the sides of each arm are angled or arced such that the far end of each arm is wider than the end closest to the intersection. The far ends of the four arms may comprise arcs, for example four arcs from a single circle, giving the cross a rounded shape. The opening 3911 in the backing layer 3910, opening 3931 in the absorbent layer 3930, and opening 3941 in the acquisition distribution layer 3940 may be aligned with the central intersection point of the cross-shaped opening 3921. The openings 3911, 3931, and 3941 may be the same size or of varying sizes.

The backing layer 3910 (as well as the backing layer of previously described embodiments) may comprise, in some embodiments, EU33 film and may optionally have a pressure-sensitive adhesive provided on a lower surface thereof. For example, the adhesive may be a water dispersible acrylic adhesive, for example K5. The adhesive may be able to be pattern spread, and may be hydrophilic.

The obscuring layer 3920 may be provided to increase patient comfort by masking the presence of wound exudate absorbed by the inner layers of the dressing. The obscuring layer 3920 may have an outer perimeter that is spaced 1 mm, or approximately 1 mm, or 0.5 mm to 3 mm, or approximately 0.5 to approximately 3 mm, beyond the adjacent perimeter edge of the dressing layer or layers provided beneath it, for example the absorbent layer 3930, ADL 3940, and/or transmission layer 3950. The obscuring layer 3920 may be provided with a plurality of viewing windows 3922 which may be used to assess the spread of exudate across the dressing 3900. The cross-shaped opening 3921 may be used as a viewing window to ascertain the level of saturation of the layer or layers underlying an attached port. The width of the cross-shaped opening 3921 may be greater than the width of an attached port to enable such assessment. Some embodiments of the obscuring layer 3920 (including other embodiments of the obscuring layer previously described) may comprise polypropylene spunbond material of suitable colors such as described above, including medical blue. Further, some embodiments of the obscuring layer 3420 may comprise a hydrophobic additive or coating.

The absorbent layer 3930 may be configured to absorb and retain exudate from a patient's wound. The absorbent layer 3930 will preferably be constructed from a material which has good absorbent qualities under negative pressure. In some embodiments (including any of the earlier described embodiments), the absorbent layer may comprise cellulose fibers or air-laid materials. Some embodiments may comprise a cellulose fibers with 40-80% superabsorbent particles (SAP), for example 40%-60% (or about 40% to about 60%) SAP or 60%-80% (or about 60% to about 80%) SAP. Heat fusible fibers can optionally be used to assist in holding the structure of the absorbent pad together. Some embodiments may combine cellulose fibers and air-laid materials, for example as a hybrid bonded airlaid composite in the range of 400-500 gsm (or about 400 to about 500 gsm), for example 460 (or about 460) gsm. The absorbent layer 3930 may include polyacrylate superabsorber powder to increase the absorbent capabilities of the material. Some embodiments of the absorbent layer 3930 comprise a tissue dispersant layer. This may, in some embodiments, be provided along the lower surface of the layer, resulting in an asymmetric construction of the absorbent layer. The tissue dispersant layer may comprise a heat fusible binder to aid in holding the layer structure together. The tissue dispersant layer may provide the advantage of enabling fluid transport. In some embodiments, the tissue dispersant layer may comprise a hot melt adhesive such as ethylene vinyl acetate (EVA), for example applied as a solution to cellulose fibers of the absorbent layer.

The adhesive layer 3970 may bond an upper surface of the acquisition distribution layer 3940 to a lower surface of the absorbent layer 3930. As illustrated, in some embodiments the adhesive layer 3970 may comprise an adhesive web or net. In other embodiments, the adhesive layer 3970 may comprise adhesive tape. Yet other embodiments may employ a hot melt adhesive, such as EVA. For example, EVA powder may be sprinkled over the ADL 3940, which may then be heat bonded to the adhesive layer 3970. In some embodiments the acquisition distribution layer 3940 and the absorbent layer 3930 may be stitched or sewn together, and the adhesive layer 3970 may comprise suitable fibers, strands, or threads. Preferred embodiments of the adhesive layer 3970 are hydrophilic so as not to affect the transport of water and/or water-based solutions between the acquisition distribution layer 3940 and absorbent layer 3930. In some embodiments, the adhesive layer may comprise a fine sprinkle of adhesive powder such that the acquisition distribution layer 3940 and absorbent layer 3930 are not bonded together across the entire upper and lower surfaces, respectively, but may be merely tacked together in a number of locations. However, some embodiments of the dressing may be constructed without the use of an adhesive between the acquisition distribution layer 3940 and absorbent layer 3930.

The acquisition distribution layer (ADL) 3940 may be constructed so as to advantageously horizontally wick fluid, such as wound exudate, as it is absorbed upward through the layers of the dressing 3900. Such lateral wicking of fluid may allow maximum distribution of the fluid through the absorbent layer 3930, enabling the absorbent layer 3930 to reach its full holding capacity. Some embodiments of the ADL 3440 (including any embodiments of the ADL previously described) may comprise cellulose in the range of 40-160 gsm (or about 40 to about 160 gsm), for example 80 (or about 80) gsm. The ADL may be constructed from a material which resists compression under the levels of negative pressure commonly applied during negative pressure therapy.

Some embodiments of the dressing 3900 may optionally comprise a spacer or transmission layer 3950. The transmission layer 3950 may comprise a porous material or 3D fabric configured to allow for the passage of fluids therethrough away from the wound site and into the upper layers of the dressing 3400. In particular, the transmission layer 3450 should remain open under the typical pressures that will be applied during negative pressure wound therapy as described above, so that the whole wound site sees an equalized negative pressure. In some embodiments, the acquisition distribution layer 3940 may be sufficient to maintain even transmission of negative pressure throughout the dressing 3900 and the transmission layer 3950 may be excluded. An outer perimeter of the transmission layer may be spaced 5 mm, or approximately 5 mm, or 2 mm to 8 mm, or approximately 2 mm to approximately 8 mm, inward of the adjacent perimeter edge of the dressing layer positioned above the transmission layer, for example the ADL 3940 or absorbent layer 3930.

The dressing 3900 may optionally comprise a wound contact layer 3960 for sealing the dressing 3900 to the healthy skin of a patient surrounding a wound area. The wound contact layer 3960 may comprise flexible polyurethane film, and may be provided with a silicone adhesive on a lower surface thereof. The wound contact layer 3960 may be perforated to allow for the transmission of fluids such as wound exudate therethrough, so that the fluids may be passed through or retained by the inner layers of the dressing 3900. Prior to use, the wound contact layer 3960 may be protected by a protective release layer 3980, which may be provided with at least one set of flaps 3981 for removing or peeling off the release layer 3980.

Further details regarding wound dressings that may be utilized with a negative pressure system, and further details regarding negative pressure systems and their methods of use are described in: PCT App. No. PCT/IB2013/002060, titled "Wound Dressing and Method of Treatment," filed July 31, 2013, U.S. Pat. No. 8,791,315, titled Systems and Methods for Using Negative Pressure Wound Therapy to Manage Open Abdominal Wounds," filed September 20, 2010, and U.S. Pat. App. No. 13/092,042, titled "Wound Dressing and Method of Use," filed April 21, 2011.

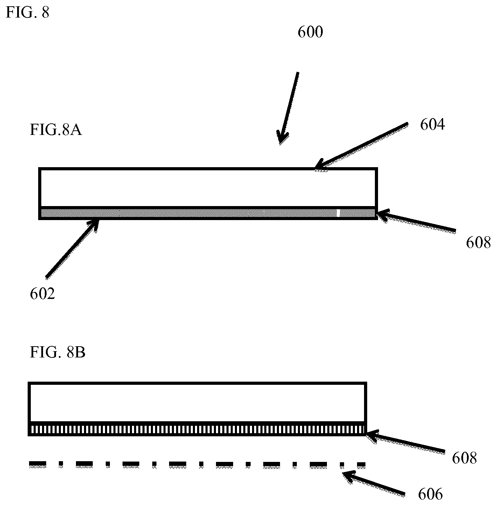

Figure 8A depicts a device 600 having a wound-contacting surface 602 and an opposing non-wound-contacting surface 604. Figure 8B depicts the device 600 *in situ* on a wound 606. The device 600 can be made of any material that is suitable for contact with the wound. Wound contact layers are known in the art and include the PROFORE wound contact non-adherent dressing (Smith and Nephew, Inc), the MEPITEL Soft Silicone Wound Contact Layer (Mölnlycke Health Care US, LLC), CUTICERIN, a low-adherent acetate gauze (Smith and Nephew, Inc) and the DRYNET Wound Veil (Smith and Nephew, Inc) . Conventionally wound contact layers are characterised by being conformable, transparent, non-adherent sheets that are placed on or in an open wound bed to protect the tissue from direct contact with other agents or dressings applied to the wound. Wound contact layers are also typically porous to allow wound exudate to pass through for absorption by an overlying, secondary dressing. Device 600 may be porous and can be a made of a non-woven, a perforated film or a mesh. Alternatively, in applications in which the device 600 is to be transiently placed into the wound to measure pH between dressing changes, the device 600 may be non-porous.

The device further includes a pH indicator 608 which is applied to one or both of surfaces 602 and/or 604. The pH indicator is immobilised on or adjacent to the surface 602 and/or 604 so that it is not washed away by the wound exudate. As described elsewhere in the Specification, the pH indicator may be utilized in combination with the dressings disclosed in Figures 1-7. In embodiments, the pH indicator may be incorporated into any of the various components disclosed in the dressings of Figures 1-7.

In embodiments, the pH indicator is chemically bound to the surface 602 and/or 604. For example, the pH indicator is covalently bound to the surface 602 and/or 604. In alternative embodiments, the surface 602 and/or 604 is provided within an adhesive and the pH indicator is covalently bound to reactive moieties within the adhesive. For example, a conventional acrylic adhesive, such as K5 (Smith & Nephew, Inc) used in the construction of wound dressings contains residues of 2-hydroxy-ethylmethacrylate, which provide a reactive functional hydroxyl (OH) group, pendant to the polymer backbone, to which the pH indicator can be covalently bound. Other suitable adhesives include acrylic-based adhesives with pendant OH or COOH groups.

In alternative embodiments, the pH indicator is physically entrapped at or adjacent to the surface. For example, the pH indicator is entrapped within a soluble microsphere, for example a microsphere made of a hydrophilic soluble polymer. Alternatively, the pH indicator is retained between layers of a soluble material, such as a polyvinyl alcohol film which is positioned adjacent to the surface.

In embodiments on which the pH indicator is only applied to one surface of a non-porous device, then an indication, for indicating which side the pH indicator is applied to may be provided. This indication allows the user to appropriately orient the device during placement on or in a wound to ensure that the surface which has the pH indicator provides the wound-contacting surface.

The pH indicator may be applied across substantially the entire surface 602 and/or 604, to allow the pH across the entire wound bed to be mapped. Alternatively, the pH indicator may be applied to discrete areas of surfaces 602 and/or 604. The pH indicator exhibits a first colour prior to contact with a wound exudate and changes colour as a function of the pH of the wound. The first colour of the pH indicator may be colourless.

The pH indicator is capable of reversibly changing colour in response to pH. In embodiments, the pH indicator is a phenylazo compound. In certain embodiments, the phenylazo compound is selected from the group listed in Table 1. In some embodiments, the phenylazo compound is not 2-[4(2-hydroxyethylsulfonyl)-phenyl]diazenyl]-4-methylphenol. In some embodiments, the phenylazo compound is not hydroxy-4-[4[(hydroxyethylsulphonyl)-phenylazo]-napthalene-2-sulphonate. In some embodiments, the phenylazo compound is not 2-fluoro-4-[4[(2-hydroxyethanesulphonyl)-phenylazo]-6-methoxy phenol. In some embodiments, the phenylazo compound is not 4-[4-(2-hydroxyethylsulphonyl)-phenylazo]-2,,6-dimethoxyphenol. In certain embodiments, the phenylazo compound is 2-[4(2-hydroxyethylsulfonyl)-phenyl]diazenyl]-4-methylphenol. The pH indicator includes a plurality of phenylazo compounds. In some embodiments, the pH indicator includes a combination of phenylazo compounds, for example a combination of phenylazo compounds selected from the group listed in Table 1. In some embodiments, the pH indicator includes a combination of two phenylazo compounds. In some embodiments, the pH indicator includes a combination of three phenylazo compounds. In some embodiments, 2-[4(2-hydroxyethylsulfonyl)-phenyl]diazenyl]-4-methylphenol is combined with at least one other phenylazo compound selected from the group listed in Table 1. The ratio of phenylazo compound may be 1:1, but other ratios are envisaged, for example, but in no way limiting, 0.5:1.5 or 1.5:0.5 or 1:2 or 2:1 or 1:0.1.

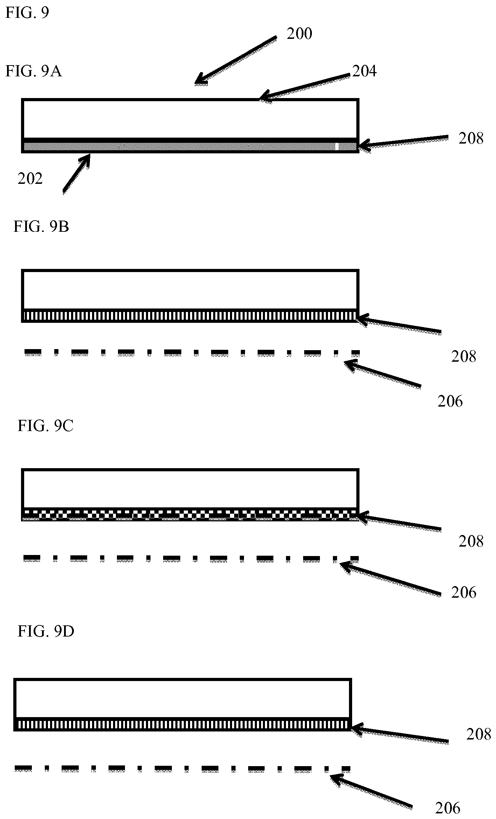

As shown in Figure 9, the device 200 comprises a pH indicating dye for indicating the pH of the wound exudate at the wound surface. Fig. 9A shows a side cross-sectional view of a device 200 which comprises a wound-contacting surface 202 and an opposing non-wound-contacting surface 204. A pH indicator 208 is provided on surface 202. The first colour of the pH indicator, prior to contact with wound exudate, may be colourless. In some embodiments, the device is an integral part of the wound dressing and functions as the wound-contacting layer. In alternative embodiments, the device is used in conjunction with a clinician's choice of a secondary dressing. For example, the non-wound-contacting surface 204 can be adhered to the wound-facing surface of a secondary dressing. Alternatively, the device 200 can be placed in or on the wound and the secondary dressing secured to device 200 using, for example, adhesive tape or adhesive film.

Fig. 9B-D illustrate changes in wound pH over time and the reversible response of the pH indicator 206. In Fig. 9B, the wound dressing has been placed on the wound 206 and the pH indicator 208 has changed colour in response to a change in pH of the wound 206 exudate. For example, if the wound exudate has pH X, the pH indicator 208 will change colour to the colour that is correlated with pH X. Because the wound dressing is *in situ* this colour change will not be visible unless the dressing is removed or the other constituent parts of dressing are transparent. In Fig. 9C, the pH of the wound exudate has altered to pH Y, and the pH indicator 208 changes colour in response to this pH change, with the colour of the pH indicator changing colour to the colour that is correlated with pH Y. Again, because the wound dressing is *in situ* this colour change will not be visible unless the dressing is removed. In Fig. 9D, the pH of the wound has reverted to pH X and the pH indicator 208, due to its reversibility, has reverted to colour X. At this point, the dressing is removed and the clinician can correlate the colour X with a pH scale and determine that the pH is pH X. This dressing thus provides a "snap shot" of the pH at the time of dressing removal. The clinician will be unaware that the pH changed to pH Y during the time that the dressing was in place.

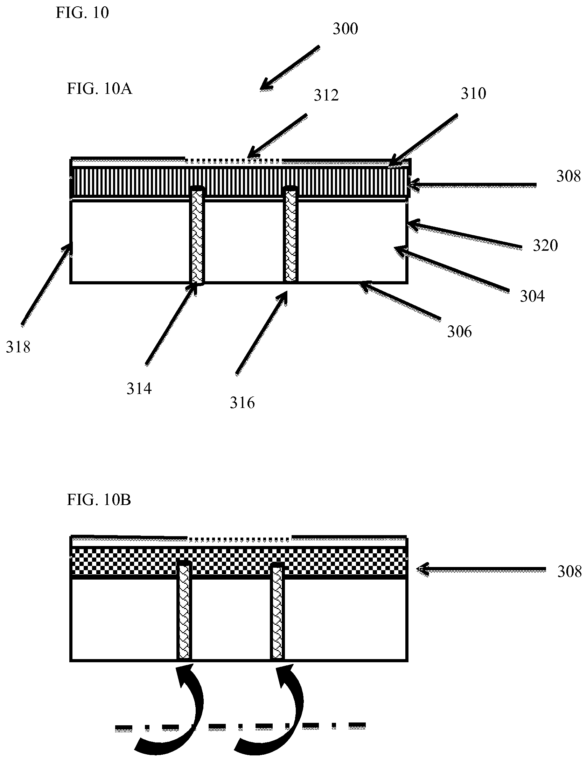

Figure 10 illustrates a wound dressing in which temporal changes in pH can be monitored whilst the dressing is *in situ* on the wound. Fig. 10A shows a side cross-sectional view of a wound dressing 300 comprising an absorbent element 304, the lower surface of which is a wound contacting surface 306. The dressing also comprises a pH indication zone 308 which is located at or adjacent to the opposing non wound-contacting surface 310. This pH indication zone includes a pH indicator (e.g., as disclosed herein) which is capable of reversibly changing colour in response to changes in pH. In this illustrated embodiment, the pH indication zone 308 is disposed above the absorbent layer 304, so the pH indicator can be monitored over time without having to remove the dressing from the patient.

A transparent layer 312 overlays at least part of the pH indication zone, which protects the integrity of the pH indicator but still allows the clinician to monitor the colour of the pH indicator over time. The dressing includes at least one conduit that is configured to direct wound exudate from the wound to the pH indication zone 308, ensuring that the pH of the wound exudate is not materially altered as it passes through the components of the wound dressing. One or a plurality of conduits could be used. As shown in Fig. 10, two conduits are used, although one or more other conduits could also be included. The two conduits 314 and 316 are oriented vertically and extend across the absorbent layer. The conduits are preferably sealed, so as not to exchange fluid with the absorbent layer, but are in communication with the pH indication zone 308 and direct the wound exudate to the pH indication zone 308 located in the upper part of the dressing. The conduits may be in the form of narrow capillaries which transmit the fluid towards the pH indication zone 308. The conduits may incorporate or may be formed from wicking materials, for example, woven, non-woven, knitted, tows or fibres made of suitable materials to facilitate wicking of the wound exudate towards the pH indication zone 308. In alternative embodiments, a pH indication zone is provided at or near a lateral edge 318 or 320 of the dressing and at least one conduit is provided within the dressing to direct the wound exudate laterally to the pH indication zone. In some embodiments, the pH indication zone is provided in a layer of the dressing which forms an outer surface of the dressing and a transparent cover layer is not used. In some embodiments, the conduits may take the form of a long strip or be of an elongated lozenge shape when viewed from the wound contact surface. Alternatively, the conduit may be formed of crosses or quadrilateral shapes. In this way it is possible to transmit wound exudate across the area of the wound to the pH indication zone.

Whilst Fig. 10 depicts a discrete pH indication zone that is provided above the absorbent element, it is envisaged that the pH indication zone can be provided in alternative locations within the dressings. It is also envisaged that the pH indication zone can exhibit additional functionality. For example, the dressing can comprise any combination of the following components; a top film, a super-absorbent layer, an absorbent layer, a spacer layer and a wound contact layer, with each component being present in the singular or plural, and the pH indication zone is or is provided within at least one of these layers. In alternative embodiments, an adhesive layer associated with at least one of these layers consists of or comprises the pH indication zone. Example wound dressing assemblies include, but are not limited to;
(a) Top film; pH indication zone; wound contacting layer;
(b) Top film; pH indication zone; spacer layer; wound contacting layer;
(c) Top film; spacer layer (= pH indication zone); wound contacting layer;
(d) Top film; pH indication zone; spacer layer; absorbent layer; wound contacting layer;
(e) Top film; pH indication zone; spacer layer; super-absorbent layer; absorbent layer, wound contacting layer;
(f) Top film; pH indication zone; spacer layer; super-absorbent layer; wound contacting layer;
(g) Top film, pH indication zone; absorbent layer; wound contacting layer;
(h) Top film, pH indication zone; super-absorbent layer; wound contacting layer.

Methods of immobilising a phenylazo dye on the devices and/or wound dressings illustrated in Figures 1-10 are also contemplated. An example includes the following steps:

In a first step, 25mg of a phenylazo pH indicating dye, for example a phenylazo pH indicating dye selected from the group listed in Table 1, is reacted with 140µl concentrated sulphuric acid for 30 mins to form a dye solution.

In a second step, 200ml of distilled water is added to the dye solution formed in the first step.

In a third step, 406 µl of a 32% w/v solution of sodium hydroxide is added to the solution formed in the second step.

In a fourth step, 25.45ml of a 2.36M solution of sodium carbonate is added to the solution formed in the third step.

In a fifth step, 1.35ml of a 32% w/v solution of sodium hydroxide is added to the solution formed in the fourth step and the volume made up to 250ml with distilled water.

In a sixth step, a material on which the pH indicating dye is to be bound is placed in the solution and left to react for approximately 1-2 hours. Examples of suitable materials include, but are not limited to: TENCEL fibres of the Durafiber product, polyurethane foam of the Allevyn product, cellulose pad of the Post-op product, or K5 adhesive-coated polyurethane film, all available from Smith & Nephew, Inc. The material is then washed with distilled water until no more dye is released. The material is then dried.

### EXAMPLES

A sample of the pad from an Opsite Post-Op dressing (Smith & Nephew, Inc) was prepared in different samples, and each sample was covalently bound with one or a combination of phenylazo dyes, selected from GJM-514, GJM-492, GJM-546, and GJM-534. The structures of these dyes are shown in Table 1. It was discovered that these dyes had colour-changing characteristics that varied according to changes in pH. The samples were covalently bound with GJM-514 alone or with GJM-514 combined with one of GJM-492, GJM-546 and GJM-534 using the method as described above in relation to Figures 1-3. The samples were exposed to buffered solutions having a pH of 5, 5.5, 6, 6.5, 7, 7.5, 8, 8.5, 9 and 9.5. Photographs were taken of each sample to demonstrate the visible changes in colour. A colour pen (for example, Dr Lange Colour Pen), a pen-type colorimeter was used to detect marginal colour changes which are undetectable by the human eye. Colour pen measurements include, but are not limited, to three different readings: the L*, a* and b* values.
- L* represents the lightness/luminosity of the colour
   o L* = 0 is black
   o L* = 100 is diffuse white
- a* is the colour's position between red/magenta and green
   ∘ A positive a* value indicates magenta
   ∘ A negative a* value indicates green
- b* is the colours position between yellow and blue
   ∘ a positive b* value indicates yellow
   ∘ a negative b* value indicates blue

### Example 1: Post-Op Pad dyed with GJM-514 (Embodiment not covered by the claimed invention)

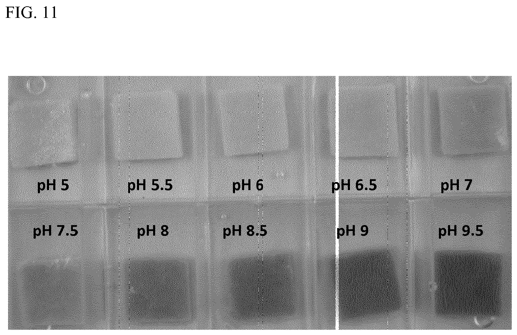

A sample of the pad from an Opsite Post-Op dressing (Smith & Nephew) covalently bound with the dye GJM- 514 was exposed to buffered solutions at pH 5 - pH 9.5. The panel of photographs in Figure 11 demonstrates the colour change of GJM-514 over this pH range, going from yellow in colour (at pH5) to pink (at pH 9.5).

Table 2 illustrates the colour pen measurements (L*, a* and b*) of the colour of the GJM-514 dye over a pH range of pH 5 - pH 9.5. An optimal dye for use as a pH indicator is one which demonstrates a linear change in a measurement of a specific parameter of colour (for example L*, a* or b*) over a broad pH range. Outside of the linear region, the dye is either unable to change colour in response to a change in pH or the change in colour is so minimal that it is undetectable.

**Table 2**

| pH | L* | a* | b* |
|---|---|---|---|
| 5 | 63.3 | -1.9 | 41.5 |
| 5.5 | 69.2 | 0.3 | 36.2 |
| 6 | 65.7 | 1.4 | 35.1 |
| 6.5 | 59.3 | 1.2 | 35.5 |
| 7 | 56.9 | 2 | 33.6 |
| 7.5 | 55.4 | 4.8 | 30.6 |
| 8 | 46.8 | 10.4 | 21.4 |
| 8.5 | 43.3 | 15.6 | 15.4 |
| 9 | 40.2 | 21.3 | 8.7 |
| 9.5 | 37.5 | 24.8 | 4.9 |

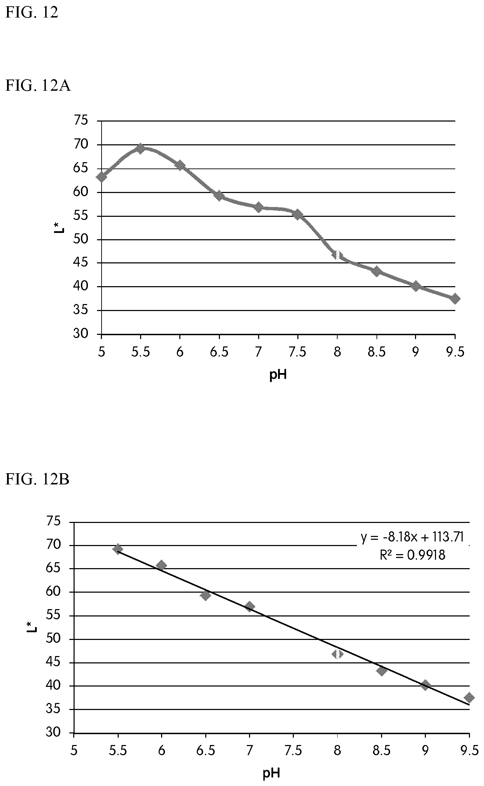

Figures 12A and 12B illustrate the L* measurements taken of the GJM-514 dye with the colour pen presented graphically. The L* results of Figure 12A show that the L* value decreases from pH 5.5 to pH 9.5 as the luminosity of the dye decreases relative to the increasing pH. These results have also been plotted in Figure 12B and demonstrate a linear region between pH 7.5 and 9.5. The trend line has a gradient of -8.18 and an R² value of 0.9918.

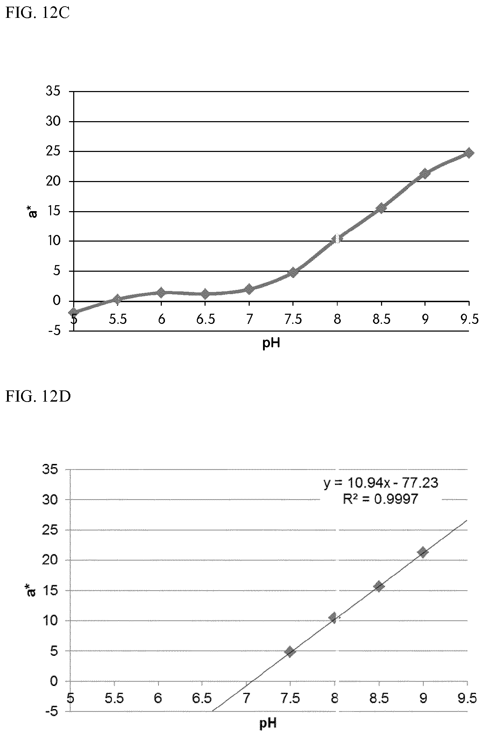

Figures 12C and 12D illustrate the a* measurements taken of the GJM-514 dye with the colour pen presented graphically. Figure 12C illustrates the a* measurements taken at various pH values between pH 5 - pH 9.5. Figure 12D illustrates the a* measurements at various pH values over the linear portion of the trend line, between pH 7.5 and 9. The trend line has a gradient of 10.94 and an R² value of 0.9997.

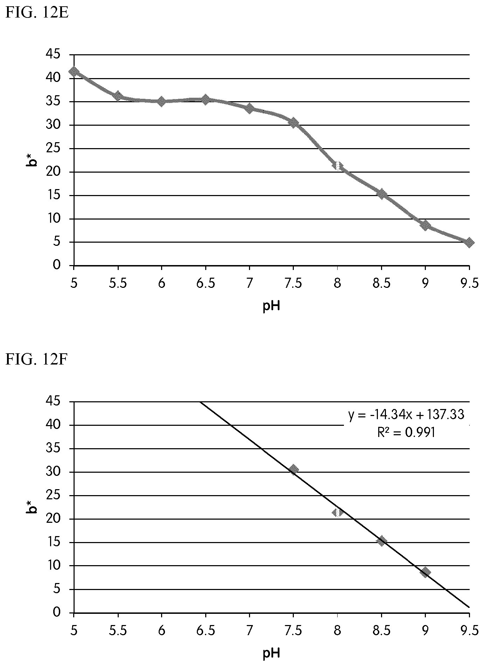

Figures 12E and 12F illustrate a graphical representation of the b* measurements taken of the GJM-514 dye. Figure 12E shows the b* measurements taken at various pH values between pH 5 - pH 9.5. Figure 12E illustrates the b* measurements at various pH values over the linear portion of a trend line. From Figure 12E it can be seen that the values are fairly consistent and steady between pH 5.5 and pH 7, and after pH 7 they start to decrease. Figure 12F shows that the results give a linear downward trend between pH 7.5 and pH 9, with a gradient of -14.34 and an R² value of 0.991.

Taking into account the colour pen results and photographs of the samples, the most accurate working range for GJM514 is between pH 7.5 and pH 9. The linear trend line of the b* measurements has a steeper gradient (-14.34) than the a* measurements (10.94) and therefore b* would be used preferentially to give a more accurate indication of the pH of the dressing when using an optical reader rather than the human eye.

### Example 2: Post-Op Pad dyed with GJM-514: GJM-492 (1:1)

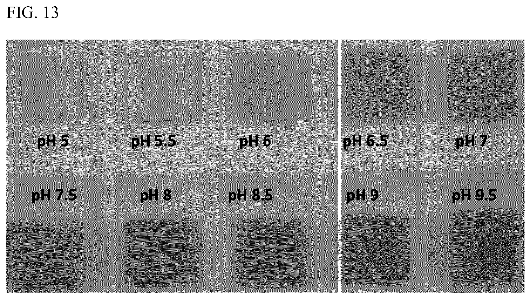

A sample of the pad from an Opsite Post-Op dressing (Smith & Nephew) covalently bound with the dye GJM- 514: GJM-492 at a 1:1 ratio was exposed to buffered solutions at pH 5 - pH 9.5. The panel of photographs in Figure 13 demonstrates the colour change over this pH range, going from yellow in colour (at pH 5) to orange in colour (at pH 9.5).

Table 3 illustrates the colour pen measurements (L*, a* and b*) of the colour of the GJM-514 : GJM-492 dye combination over a pH range of pH 5 - pH 9.5.

**Table 3**

| pH | L* | a* | b* |
|---|---|---|---|
| 5 | 53.8 | 11.5 | 43.3 |
| 5.5 | 50.7 | 17.4 | 37.9 |
| 6 | 45.3 | 23.9 | 37.5 |
| 6.5 | 40.4 | 29.9 | 35.4 |
| 7 | 39.7 | 30.9 | 33.8 |
| 7.5 | 39.9 | 30.4 | 29.9 |
| 8 | 34.5 | 31.5 | 29.2 |
| 8.5 | 37.4 | 28 | 29.3 |
| 9 | 33.8 | 30.7 | 25 |
| 9.5 | 33.1 | 31.3 | 23.2 |

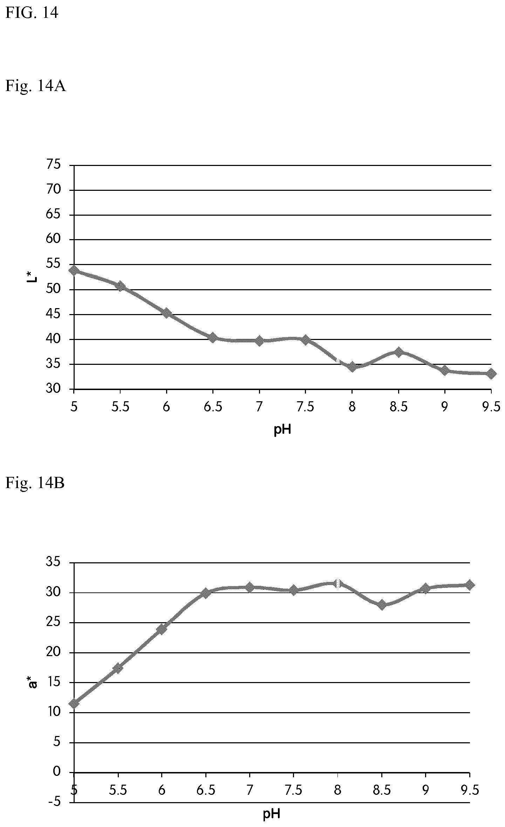

Figure 14A illustrates the L* measurements taken with the colour pen presented graphically. The L* results presented in Figure 14A show that the value for L* decreases over the range of pH 5.5 to pH 9.5 but does not follow a linear downward trend. The L* value is therefore not considered to be a reliable indicator of the colour change of this dye combination over the pH range tested.

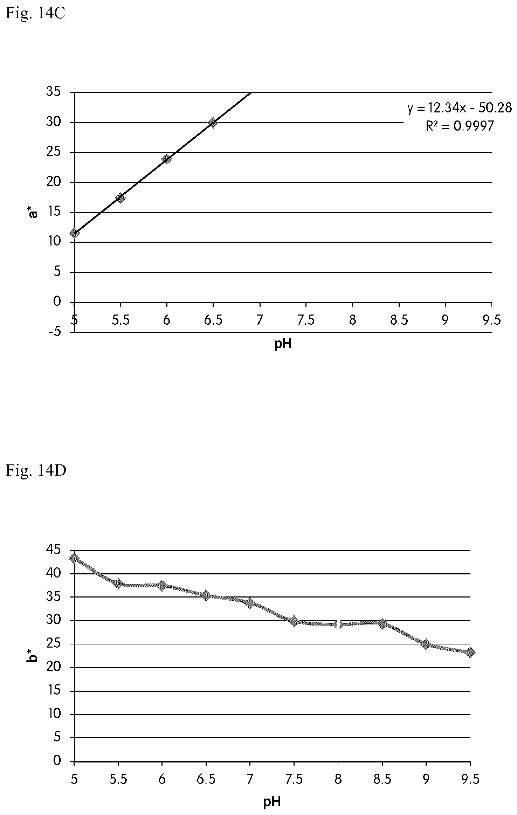

Figures 14B and 14C illustrate the a* measurements taken with the colour pen presented graphically. Figure 14B illustrates the a* measurements taken at various pH values between pH 5 - pH 9.5. Figure 14C illustrates the a* measurements at various pH values over the linear portion of a trend line. An upwardly linear trend (gradient = 12.34, R² = 0.9997) is identifiable between pH 5 and 6.5, demonstrating that there is a detectable change in colour along the red/magenta to green scale over this pH range.

Figure 14D illustrates a graphical representation of the b* measurements taken with the colour pen. It can be seen that there is not a significant change in b* value, but there is a downwards trend.

Taking into account the colour pen results and photographs of the samples, the working range for this dye combination appears to be between pH 5 and pH 6.5. With a* giving a useable trend line for this region that could be used to estimate the pH from the material colour.

### Example 3: Post-Op Pad dyed with GJM-514: GJM-546 (1:1)

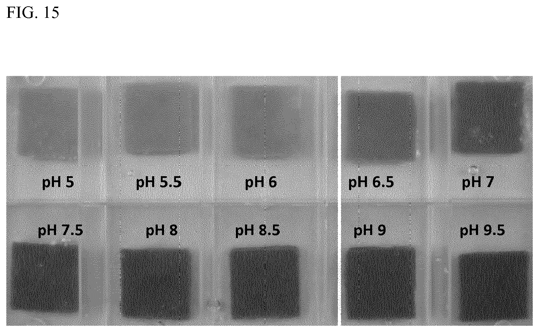

A sample of the pad from an Opsite Post-Op dressing (Smith & Nephew) covalently bound with the dye GJM 514:546 at a 1:1 ratio was exposed to buffered solutions at pH 5 - pH 9.5. The panel of photographs in Figure 15 demonstrates the colour change over this pH range, going from orange in colour (at pH 5) to pink (at pH 9.5).

Table 4 illustrates the colour pen measurements (L*, a* and b*) of the colour of the GJM-514 : GJM-546 dye combination over a pH range of pH 5 - pH 9.5.

**Table 4**

| pH | L* | a* | b* |
|---|---|---|---|
| 5 | 45.7 | 22.7 | 44.1 |
| 5.5 | 43.4 | 22.8 | 40.1 |
| 6 | 43.9 | 24.8 | 34.6 |
| 6.5 | 36.5 | 27 | 25 |
| 7 | 33.4 | 25.7 | 16 |
| 7.5 | 28.3 | 27.8 | 7.1 |
| 8 | 26.9 | 26.6 | 1.3 |
| 8.5 | 25.6 | 29.3 | -0.7 |
| 9 | 24.5 | 28.8 | -2.3 |
| 9.5 | 23.9 | 29.5 | -3.8 |

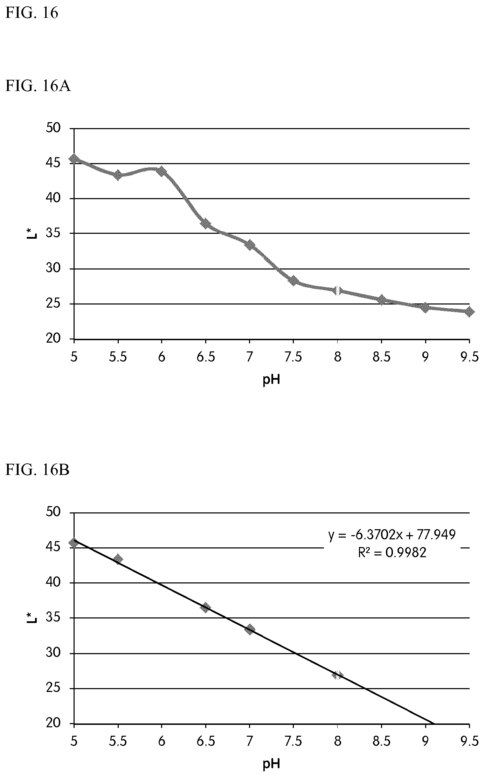

Figures 16A and 16B illustrate a graphical representation of the L* measurements taken with the colour pen. Figure 16A shows all data points whilst Figure 16B is a re-plot of the data points in the linear region between pH 5 to pH 8. The trend line has a gradient of -6.3702 with an R² value of 0.9982.

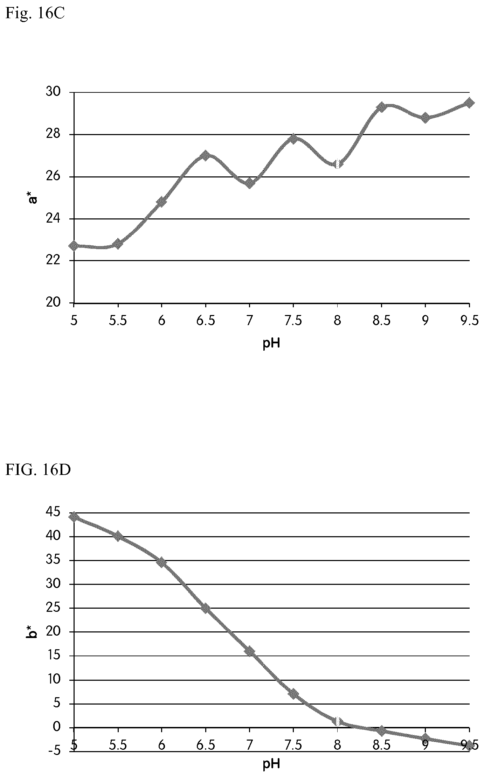

Figure 16C illustrates the a* measurements taken with the colour pen presented graphically over the pH 5 - pH 9.5 range. The results are too variable for the a* measurement to be considered of use in reliably measuring a colour change in the GJM 514:546 dye combination in response to changes in pH.

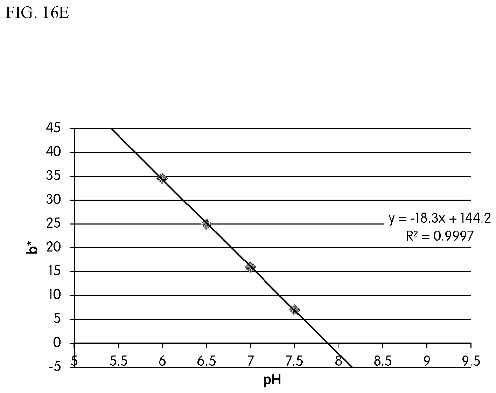

Figures 16D and 16E illustrate a graphical representation of the b* measurements taken with the colour pen. Figure 16E shows the b* measurements taken at various pH values between pH 5 - pH 9.5 and it can be seen that the results follow a downward trend from pH 5 to pH 8, but it appears to plateau after pH 8. Figure 16E illustrates the b* measurements at various pH values over the linear portion of a trend line which has a gradient of -18.3 and an R² of 0.9997. As the b* results gave a steeper gradient it is believed that monitoring the b* value would give a more accurate reading of the pH from the dressing colour. The working range for this dye combination appears to be pH 6 to pH 7.5.

### Example 4: Post-Op Pad dyed with GJM 514:534 (1:1)

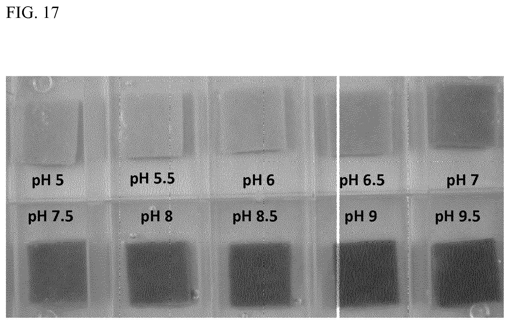

A sample of the pad from an Opsite Post-Op dressing (Smith & Nephew) impregnated with the dye GJM 514:534 at a 1:1 ratio was exposed to buffered solutions at pH 5 - pH 9.5. The panel of photographs in Figure 17 demonstrates the colour change over this pH range, going from yellow in colour (at pH 5) to red in colour (at pH 9.5).

Table 5 illustrates the colour pen measurements (L*, a* and b*) of the colour of the GJM-514:GJM-534 dye combination over a pH range of pH 5 - pH 9.5

**Table 5**

| pH | L* | a* | b* |
|---|---|---|---|
| 5 | 53.4 | 6.1 | 50.3 |
| 5.5 | 52.3 | 7.5 | 45.4 |
| 6 | 53.8 | 7.6 | 46.1 |
| 6.5 | 49.7 | 9.8 | 35.4 |
| 7 | 43.1 | 16.2 | 29.9 |
| 7.5 | 37.4 | 16.2 | 18.9 |
| 8 | 33.4 | 20.4 | 11.9 |
| 8.5 | 31.9 | 22.8 | 5.3 |
| 9 | 27.7 | 27.6 | 3.6 |
| 9.5 | 28.9 | 29.1 | -0.5 |

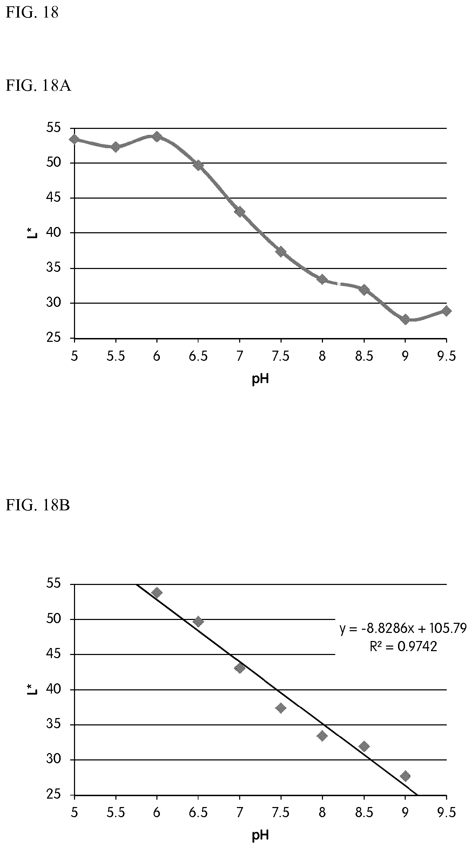

Figures 18A and 18B illustrate a graphical representation of the L* measurements taken with the colour pen. Figure 18A shows all data points whilst Figure 18B shows only those data points in the linear region. A general downward trend from pH 6 to pH 9 is observed. The trend line has a gradient of -8.8286 and an R² value of 0.9742.

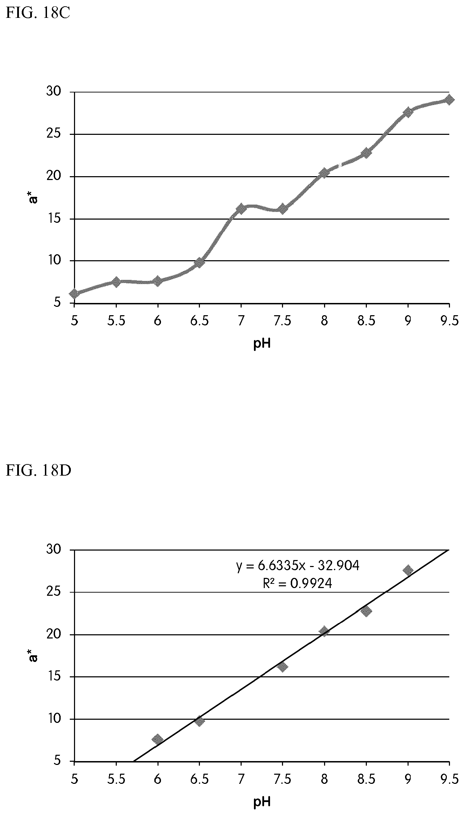

Figures 18C and 18D illustrate the a* measurements taken with the colour pen presented graphically. Figure 18C illustrates the a* measurements taken at various pH values between pH 5 - pH 9.5. Figure 18D illustrates the a* measurements at various pH values over the linear portion of a trend line. The results demonstrate an upwards trend between pH 6 to pH 9, with the trend line having a gradient of 6.6335 and an R² value of 0.9924.

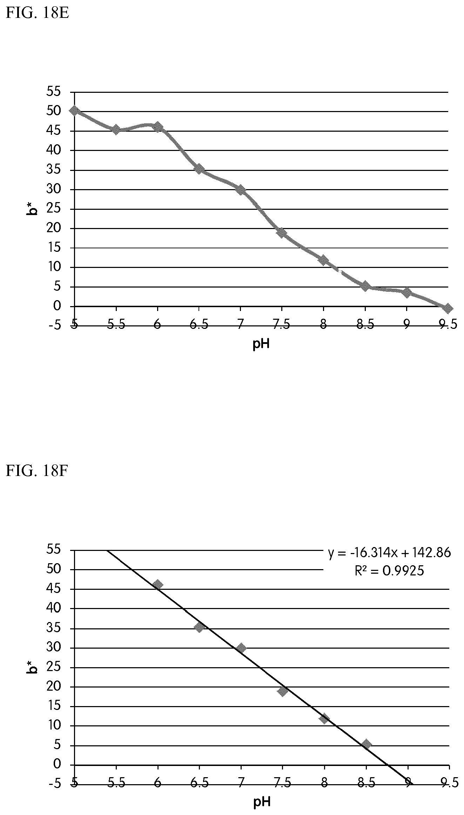

Figures 18E and 18F illustrate a graphical representation of the b* measurements taken with the colour pen. Figure 18E shows the b* measurements taken at various pH values between pH 5 - pH 9.5 and it can be seen that the results follow a downward trend until pH 9. The trend line illustrated in Figure 18F has a gradient -16.314 and an R² value of 0.9925 between pH 6 and pH9. From the colour pen measurements the working range of this dye combination is between pH 6 and pH 9, and the b* value could be used to accurately measure the pH from the material colour.

### Example 5: Post-Op Pad dyed with GJM 514:534 (1:0.509)

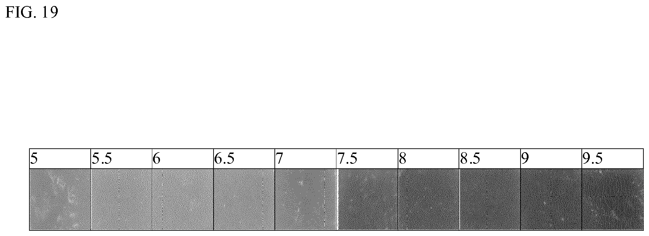

A sample of the pad from an Opsite Post-Op dressing (Smith & Nephew) covalently bound with the dye GJM 514:534 at a 1:0.509 ratio was exposed to buffered solutions at pH 5 - pH 9.5. The panel of photographs in Figure 19 demonstrates the colour change over this pH range, going from yellow in colour (at pH 5) to red in colour (at pH 9.5).

Table 6 illustrates the colour pen measurements (L*, a* and b*) of the colour of the GJM-514:GJM-534 dye combination over a pH range of pH 5 - pH 9.5

**Table 6**

| pH | L* | a* | b* |
|---|---|---|---|
| 5 | 55.4 | 4.9 | 43.1 |
| 5.5 | 57.6 | 2.9 | 42.6 |
| 6 | 56.8 | 3.4 | 42.7 |
| 6.5 | 51.2 | 5 | 40 |
| 7 | 49 | 8.8 | 34.7 |
| 7.5 | 39.8 | 11.4 | 23.5 |
| 8 | 39 | 17.6 | 15 |
| 8.5 | 36.5 | 22.4 | 10.1 |
| 9 | 34.2 | 24.3 | 5.8 |
| 9.5 | 32.3 | 25.3 | 0.3 |

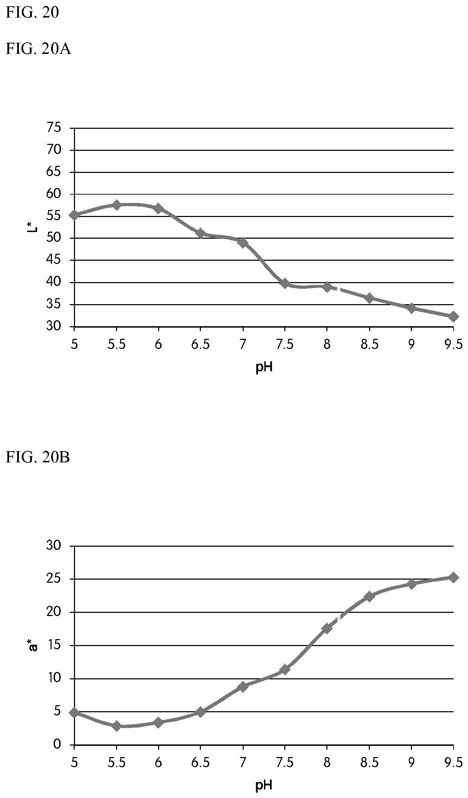

Figures 20A illustrates a graphical representation of the L* measurements taken with the colour pen. A general downward trend from pH 6 to pH 9.5 is observed.

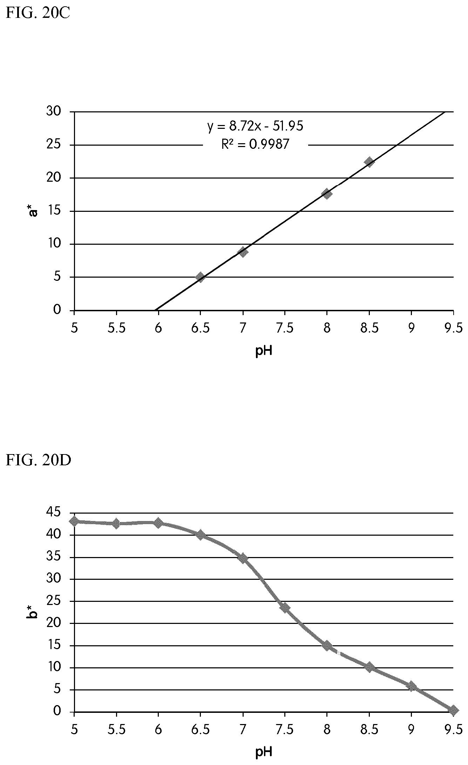

Figures 20B and 20C illustrate the a* measurements taken with the colour pen presented graphically. Figure 20B illustrates the a* measurements taken at various pH values between pH 5 - pH 9.5. Figure 20C illustrates the a* measurements at various pH values over the linear portion of a trend line. The results demonstrate a linear upwards trend between pH 6.5 to pH 8.5, with the trend line having a gradient of 8.72 and an R² value of 0.9987.

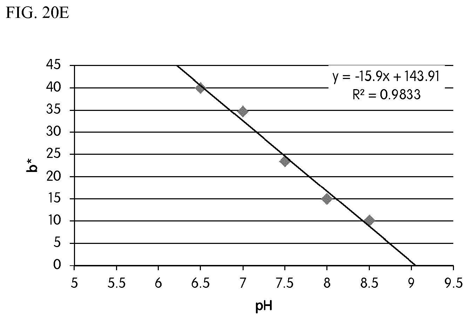

Figures 20D and 20E illustrate a graphical representation of the b* measurements taken with the colour pen. Figure 20D shows the b* measurements taken at various pH values between pH 5 - pH 9.5 and it can be seen that the results follow a downward trend between pH 6 and pH 8.5. The trend line illustrated in Figure 20E has a gradient 15.9 and an R² value of 0.9833. Taking into account the colour pen results and the photographs of the samples, the working range of this dye combination is between pH 6 and pH 8.5, and the b* value could be used to accurately measure the pH from the material colour.

### Examples 6 and 7

Further to the above general method for preparing covalently bonded dye, different materials were also used to which to bind the dye.

A sample of a gauze (Kerlix Trademark of Covidiene) and a polyvinyl alcohol foam (V.A.C. WhiteFoam, trade mark of KCI) were covalently bound with the dye GJM-546 and 492 in a ratio 1:3.92, as decribed throughout this disclosure.

These later materials can be used as pH sensing fillers for Negative Pressure Wound Therapy (NPWT). They were evaluated by use of the following models and experiments.

### Materials

| **Material** |
|---|
| Pork Meat (loin or shoulder 2kg approx. Intact skin and a surface area 20x20cm approx.) |
| pH sensitive VAC foam |
| pH sensitive gauze |
| Renasys drapes |
| Horse serum |
| Citric Acid |
| Sodium Bicarbonate |

### Equipment

| **Equipment** |
|---|
| Renasys EZ plus pump |
| Peristaltic pump |
| Renasys EZ canister |
| Epidural needle |
| Clingfilm |
| Tubing |
| Glass Dish |
| Scalpel |
| pH meter |

### Method

Use these solutions to adjust horse serum to pH 5 and pH 8, for use in the meat mode.
1. Place a sheet of cling film in the bottom of a glass dish/tray and place a piece of pork with intact skin upwards on the cling film.
2. Wrap the meat in the cling film and add more if necessary so that the meat is completely sealed.
3. Using a scalpel create 2 wounds each approximately 50mm in diameter and 25mm deep in the tissue (and at least 2cm apart), by removing the skin/fat/muscle, with a relatively flat bottom and minimal tissue flaps.
4. Insert an epidural catheter needle through the side of the wound so that the tip appears at the outside edge of the meat. Use the needle to feed the peristaltic pump tubing through so that it lies at the base of the wound. (Repeat for the other wound).
5. Using small pieces of Flexi-fix and/or adhesive putty ("white-tac") secure and seal the openings where the fluid tubes exit the cling film.
6. The following combinations are to be tested:
   a. Dyed VAC foam
   b. Dyed gauze
7. Add foam to bridge onto intact healthy skin and link both bridges together to work from a single port. Seal over the wounds, fillers and bridging foam with drapes.
8. Make a small hole in the drape where it lies over a foam bridge and attach a port using Flexi-fix strips.
9. Connect the port to a RENASYS NPWT pump (set at -120mmHg) and switch on.
10. Turn on the peristaltic pump (set to deliver 40 µl/min) to deliver fluid to the wound bed of horse serum at pH 8.
11. Monitor the dressings until fluid starts to appear in the canister (make a note of the length of time)
12. Change the fluid to horse serum at a pH of 5, and leave to flow for the amount of time determined in step 11). Then take a photograph of the dressings.
13. Change the fluid to horse serum at a pH of 8, and leave to flow for the amount of time determined in step 11). Then take a photograph of the dressings.
14. Change the fluid back to horse serum at a pH of 5, and leave to flow for the amount of time determined in step 11). Then take a photograph of the dressings.
15. At the end of the experiment disconnect the tubing and seal the meat in cling film for disposal. Clean all surfaces that had contact with the meat with soap/water.

Determination of the ability of dyed VAC foam and gauze to detect changes in pH of wound fluid.

The pH sensitive gauze and VAC foam were washed after the first meat model experiment and then used in an additional wound model, with pH adjusted water. In addition the extra piece of pH sensitive dyed gauze was placed in a clear Perspex wound model and fluid pumped through.

All wound models were monitored by taking photographs, those carried out in meat could only be monitored from the top surface, but the clear Perspex model could be monitored from all sides.

### Results and Discussion

The foam was orange in colour when it was loaded into the wound, but the gauze was more of a red colour. It is believed the gauze is red in colour due to the presence of PHMB on the gauze which would make it basic.

### Meat Model 1

The experiment was started by pumping pH 5 horse serum through into the wound filler for approximately 2.5 hours before fluid started to appear in the canister and the material started to change colour. After approximately 5.5 hours the pH 5 horse serum solution was changed to pH 8 horse serum and this was run overnight. In the morning the solution was then changed back to pH 5 horse serum and was pumped in for several hours (due to time restrictions the flow rate was increased to 80µl/min after 3.5 hours).

The images of the pH sensitive dyed gauze changing over time can be seen in Figures 21 A to F; showing that the gauze had started to go orange after 5.5 hours of exposure to pH 5 horse serum and after a night of exposure to pH 8 serum the gauze had returned to a red colour. Then after several hours of exposure to pH 5 the gauze was starting to turn orange again at which time the experiment was ended. Upon removal of the gauze it could be seen that the bottom of the gauze was mostly orange and it could be seen that the colour and therefoe the pH were changing through the gauze in a direction from the wound bed towards the drape, which can be explained by the fact that the wound tends to fill up like the filling of a bath and therefore the pH takes time to change from one pH to the other as the pump fluid is slowly transported through the wound filler.

Images of the pH sensitive dyed VAC foam changing over time can be seen in Figures 22 A to F They show that the foam had gone yellow when exposed to pH 5 horse serum (5.5 hours image), and that when exposed to pH 8 overnight the foam went red. As with the gauze the foam had started to turn yellow/orange after re-exposure to pH 5 serum for several hours before the experiment was ended, the yellow/orange colour can most clearly be seen near the bridging foam.

### Meat Model 2

For the second meat model the basic aqueous solution was used first and was left pumping into the model overnight. The next morning the solution was then changed to an acidic aqueous solution and left pumping for several hours.

The images for the pH sensitive gauze can be seen in Figures 23A to F and show that the gauze went red in colour in basic solution and within 5 hours of the fluid being switched to acidic aqueous solution the gauze had started to turn orange. It is believed that this colour change will originate at the base of the wound and work its way up to the surface as the pH in the wound changes, which as mentioned earlier would be similar to the way in which a bath fills up. It is clear the colour change on the surface starts near the area directly below the port, can be explained as this is the destination (exit point) of the fluid and so the pH would stabilise around this area on the surface first.

The same trend is seen with the dyed VAC foam, as shown in Figures 24A to F The foam turns red when in the presence of basic fluid and the when the fluid is changed to acidic the foam starts to turn yellow in colour. Like the gauze the colour change seen on the surface is first noticeable around the port where the fluid is removed from the wound.

### Clear Perspex Wound Model

The experiment was also carried out using the pH sensitive dyed gauze in a clear Perspex wound model to be able to visualise the colour change throughout the wound. The fluid was not pumped in from the bottom on this occasion but from the left hand side of the wound as seen on the images in Figures 25 A to H. The fluid inlet is on the same side as the port and halfway up the wound wall. It is believed that the area of this wound is smaller than those created in the meat, hence the colour change occurring faster as the pump speed is the same in both experiments. It can be seen that as the basic fluid is pumped into the wound the gauze turns red (at T=0 hours there was already some basic fluid in the wound hence part of the gauze already being red in colour). It can be seen from all the images in Figures 25 A to H, both the top surface of the wound (top image) and the bottom (bottom image of each pair), that the colour change moves across the wound from left to right and that the bottom of the wound is slightly ahead of the upper surface of the wound. This colour change is as expected, as fluid fills up from the bottom and so the pH changes at the bottom before the top. The Perspex model is not as realistic as the meat model as the fluid and content from the meat would mean that the pH could take longer to change due to possible buffering effects.

### Conclusions and Recommendations

Both the pH sensitive dyed VAC foam and gauze, changed colour as they were exposed to different pH solutions. The colours for indicating the different pH's were clearly visible, and the colour could be reversed by addition of the other pH solution to the wound.

It is to be understood that the foregoing description is merely illustrative and is not to be limited to the details given. While several embodiments have been provided in the present disclosure, it should be understood that the disclosed devices and method and their components, may be embodied in many other specific forms without departing from the scope of the disclosure.

Variations and modifications will occur to those of skill in the art after reviewing this disclosure. The disclosed features may be implemented, in any combination and sub-combinations (including multiple dependent combinations and sub-combinations), with one or more other features described herein. The various features described or illustrated above, including any components thereof, may be combined or integrated in other systems. Moreover, certain features may be omitted or not implemented.

## Claims

1. A device for determining the pH of a wound exudate comprising a surface configured to contact the wound and a pH indicator applied to the surface, wherein the pH indicator has a first colour prior to contact with the wound exudate and changes colour as a function of the pH of the wound exudate,
wherein the surface comprises an adhesive comprising the pH indicator, and
wherein the pH indicator comprises a combination of phenylazo compounds.

2. The device according to claim 1, wherein the pH indicator is immobilized on or adjacent to the surface.

3. The device according to claim 1, wherein the pH indicator is covalently bound to the surface.

4. The device according to claim 1, wherein the pH indicator is provided within the adhesive and wherein the pH indicator is covalently bound to reactive moieties within the adhesive.

5. The device according to any preceding claim, wherein one or more of the phenylazo compounds are selected from the group consisting of:
2-[4(2-hydroxyethylsulfonyl)- phenyl]diazenyl]-4-methylphenol;
1 -hydroxy-4 - [4 [(hy droxyethylsulphony 1) - phenylazo]-napthalene-2-sulphonate;
2-fluoro-4-[4[(2-hydroxyethanesulphonyl)- phenylazo]-6-methoxy phenol; and
4-[4-(2-hydroxyethylsulphonyl)-phenylazo]- 2,,6-dimethoxyphenol.

6. The device according to any of claims 1 to 5, wherein the device is a conformable non-woven, mesh or perforated film.

7. The device according to any of claims 1 to 6, wherein the pH indicator is applied to the entire wound-contacting surface.

8. The device according to any of claims 1 to 6, wherein the pH indicator is applied to a discrete area of the wound-contacting surface.

9. The device according to claims 1 to 8, wherein the device is used in combination with a secondary dressing.

10. The device according to claim 9, wherein the device is the wound contacting layer of a wound dressing.

11. A wound dressing comprising the device of any of claims 1 to 8.

## Patentansprüche

1. Vorrichtung zum Bestimmen des pH-Werts eines Wundexsudats, umfassend eine Oberfläche, die zum Kontakt mit der Wunde gestaltet ist, und einen pH-Indikator, der an der Oberfläche aufgebracht ist, wobei der pH-Indikator eine erste Farbe vor Kontakt mit dem Wundexsudat aufweist und die Farbe als eine Funktion des pH-Werts des Wundexsudats ändert,
wobei die Oberfläche einen Klebstoff umfasst, der den pH-Indikator umfasst, und
wobei der pH-Indikator eine Kombination von Phenylazoverbindungen umfasst.

2. Vorrichtung nach Anspruch 1, wobei der pH-Indikator auf oder benachbart zu der Oberfläche immobilisiert ist.

3. Vorrichtung nach Anspruch 1, wobei der pH-Indikator kovalent an die Oberfläche gebunden ist.

4. Vorrichtung nach Anspruch 1, wobei der pH-Indikator innerhalb des Klebstoffs bereitgestellt ist und wobei der pH-Indikator kovalent an reaktive Einheiten in dem Klebstoff gebunden ist.

5. Vorrichtung nach einem der vorstehenden Ansprüche, wobei eine oder mehrere der Phenylazoverbindungen ausgewählt sind aus der Gruppe bestehend aus:
2-[4(2-Hydroxyethylsulfonyl)phenyl]diazenyl]-4-methylphenol;
1-Hydroxy-4-[4[(hy droxyethylsulfony 1)-phenylazo]naphthalin-2-sulfonat;
2-Fluor-4-[4[(2-hydroxyethansulfonyl)-phenylazo]-6-methoxyphenol; und
4-[4-(2-Hydroxyethylsulfonyl)phenylazo]-2,6-dimethoxyphenol.

6. Vorrichtung nach einem der Ansprüche 1 bis 5, wobei die Vorrichtung ein formbares bzw. formbarer Vlies, Netz oder perforierter Film ist.

7. Vorrichtung nach einem der Ansprüche 1 bis 6, wobei der pH-Indikator auf die gesamte Wundkontaktfläche aufgebracht ist.

8. Vorrichtung nach einem der Ansprüche 1 bis 6, wobei der pH-Indikator auf einen diskreten Bereich der Wundkontaktfläche aufgebracht ist.

9. Vorrichtung nach den Ansprüchen 1 bis 8, wobei die Vorrichtung in Kombination mit einem Sekundärverband verwendet wird.

10. Vorrichtung nach Anspruch 9, wobei die Vorrichtung die Wundkontaktschicht eines Wundverbandes ist.

11. Wundverband umfassend die Vorrichtung nach einem der Ansprüche 1 bis 8.

## Revendications

1. Dispositif destiné à déterminer le pH d'un exsudat de plaie comprenant une surface conçue pour venir en contact avec la plaie et un indicateur de pH appliqué à la surface, l'indicateur de pH ayant une première couleur avant le contact avec l'exsudat de plaie et changeant de couleur en fonction du pH de l'exsudat de plaie,
la surface comprenant un adhésif comprenant l'indicateur de pH, et
l'indicateur de pH comprenant une combinaison de composés phénylazoïques.

2. Dispositif selon la revendication 1, dans lequel l'indicateur de pH est immobilisé sur ou adjacent à la surface.

3. Dispositif selon la revendication 1, dans lequel l'indicateur de pH est lié de façon covalente à la surface.

4. Dispositif selon la revendication 1, dans lequel l'indicateur de pH est disposé à l'intérieur de l'adhésif et dans lequel l'indicateur de pH est lié de façon covalente à des groupements réactifs à l'intérieur de l'adhésif.

5. Dispositif selon une quelconque revendication précédente, dans lequel un ou plusieurs des composés phénylazoïques sont choisis dans le groupe constitué par :
le 2-[4(2-hydroxyéthylsulfonyl)-phényl]diazényl]-4-méthylphénol ;
le 1-hydroxy-4-[4[(hydroxyéthylsulfony 1)-phénylazol-naphtalène-2-sulfonate ;
le 2-fluoro-4-[4[(2-hydroxyéthanesulfonyl)-phénylazo]-6-méthoxyphénol ; et
le 4-[4-(2-hydroxyéthylsulfonyl)-phénylazo]-2,6-diméthoxyphénol.

6. Dispositif selon l'une quelconque des revendications 1 à 5, le dispositif étant un non-tissé, filet ou film perforé souple.

7. Dispositif selon l'une quelconque des revendications 1 à 6, dans lequel l'indicateur de pH est appliqué à la totalité de la surface de contact avec la plaie.

8. Dispositif selon l'une quelconque des revendications 1 à 6, dans lequel l'indicateur de pH est appliqué à une zone discrète de la surface de contact avec la plaie.

9. Dispositif selon les revendications 1 à 8, le dispositif étant utilisé en combinaison avec un pansement secondaire.

10. Dispositif selon la revendication 9, le dispositif étant la couche de contact avec la plaie d'un pansement.

11. Pansement comprenant le dispositif de l'une quelconque des revendications 1 à 8.
